# EUROPEAN PATENT SPECIFICATION

(11) **EP 0 797 630 B1**
(45) Date of publication and mention of the grant of the patent: **31.01.2001**
(21) Application number: 95942524.0
(22) Date of filing: 30.11.1995
(51) Int. Cl.: C09D 1/00, C09D 5/00

(54) **COATING COMPOSITION HAVING ANTI-REFLECTIVE AND ANTI-FOGGING PROPERTIES**
BESCHICHTUNG MIT ANTI-REFLEKTIERENDEN UND BESCHLAGSVERHINDERNDEN EIGENSCHAFTEN
COMPOSITION DE REVETEMENT POSSEDANT DES PROPRIETES ANTIREFLET ET ANTI-BUEE

(30) Priority: 12.12.1994 US 354242
(43) Date of publication of application: 01.10.1997
(73) Proprietor: MINNESOTA MINING AND MANUFACTURING COMPANY, St. Paul, Minnesota 55133-3427 (US)
(72) Inventor: SCHOLZ, Matthew, T., Saint Paul, MN 55133-3427 (US); KAUSCH, William, L., Saint Paul, MN 55133-3427 (US); BOSTON, David, R., Saint Paul, MN 55133-3427 (US); ZOBOROWSKI, Joseph, M., Saint Paul, MN 55133-3427 (US)
(74) Representative: VOSSIUS & PARTNER
(86) International application number: US9515648
(87) International publication number: WO9618691

(56) References cited:
- DATABASE WPI Week 9315 Derwent Publications Ltd., London, GB; AN 93121493[15] & JP,A,05 059 203 (NIPPON CARBIDE AND SHOKUBAI KASEI) , 9 March 1993
- DATABASE WPI Week 9315 Derwent Publications Ltd., London, GB; AN 93121576[15] & JP,A,05 059 300 (SHOKUBAI KASEI) , 9 March 1993

## Description

This invention relates generally to coating compositions and methods for producing optically clear articles with very low reflection and exceptional anti-fogging properties even under high humidity conditions. Such properties are desirable in articles such as face shields used for personal protection, ophthalmic lenses, architectural glazings, windows, automotive windshields and the like.

There are numerous instances where optically clear articles would be enhanced if the tendency of the articles to cause glare or to be obscured by the formation of a fog on a surface of the article could be reduced. For example, protective eyewear (goggles, face shields, helmets, etc.), ophthalmic lenses, architectural glazings, decorative glass frames, motor vehicle windows and windshields may all reflect light in a manner that causes an annoying and disruptive glare. Use of such articles may also be detrimentally affected by the formation of a moisture vapor fog on a surface of the article.

Glare is the undesirable reflection of light from a surface upon which the light is incident. In general, glare may be reduced by increasing the amount of light transmitted by the article, thereby reducing the amount of light which is available for reflection.

Alternatively, the article surface can be modified (e.g., roughened, embossed, etc.) to cause the light to be reflected from the article more randomly and, therefore, with less glare.

Coatings which significantly increase the percent transmission of light and provide articles having very low reflection ("anti-reflective coatings") are known in the art. For example, U.S. Patent No. 4,816,333 to Lange et al. (also assigned to 3M) discloses anti-reflective coatings of silica particles. The coating solution contains colloidal silica particles and optionally a surfactant ("Triton™ X-100" and "Tergitol TMN-6") to improve the wettability of the coating solution. U.S. Patent No. 4,374,158 (Taniguchi et al.) discloses an anti-reflective coating using a gas phase treatment technique. The coating may optionally contain additives as surface controlling agents, such as silicone type surfactants. Various other types of anti-reflective coatings are disclosed in U.S. Patent Nos. 2,366,516; 3,301,701; 3,833,368; 4,190,321, 4,271,210; 4,273,826; 4,346,131 and 4,409,285; by Cathro et al. in "Silica Low-Reflection Coatings for Collector Covers by a Dye-Coating Process," Solar Energy, Vol. 32, No. 5, pp. 573-579 (1984); and by J.D. Masso in "Evaluation of Scratch Resistant and Anti-reflective Coatings for Plastic Lenses," Proceedings of the 32nd Annual Technical Conference of the Society of Vacuum Coaters, Vol. 32, p. 237-240 (1989). None of these anti-reflective coatings produce a durable anti-fog coating.

In general, fog formation occurs under conditions of high humidity and high temperature or at interfacial boundaries where there is a large temperature and humidity difference. Coatings which reportedly reduce the tendency for surfaces to "fog up" (i.e., anti-fogging coatings) are known. For example, U.S. Patent No. 3,212,909 to Leigh discloses the use of ammonium soap, such as alkyl ammonium carboxylates in admixture with a surface active agent which is a sulfated or sulfonated fatty material, to produce an anti-fogging composition. U.S. Patent No. 3,075,228 to Elias discloses the use of salts of sulfated alkyl aryloxypolyalkoxy alcohol, as well as alkylbenzene sulfonates, to produce an anti-fogging article useful in cleaning, and imparting anti-fog properties to various surfaces. U.S. Patent No. 3,819,522 to Zmoda, discloses the use of surfactant combinations comprising derivatives of decyne diol as well as surfactant mixtures which include ethoxylated alkyl sulfates in an anti-fogging window cleaner surfactant mixture.

Japanese Patent Kokai No. Hei 6[1994]-41335 discloses a clouding and drip preventive composition comprising colloidal alumina, colloidal silica and an anionic surfactant.

U.S. Patent No. 4,478,909 (Taniguchi et al.) discloses a cured anti-fogging coating film which comprises polyvinyl alcohol, a finely divided silica, and an organic silicon compound, the carbon/silicon weight ratio apparently being important to the film's reported anti-fogging properties. Various surfactants, including fluorine-containing surfactants, may be used to improve the surface smoothness of the coating.

Other anti-fog coatings incorporating surfactants are described in U.S. Patents 2,803,552; 3,022,178 and 3,897,356. "Anti-fog Antistat Eases Processing Problems," Modern Plastics, Oct. 1988, discusses antistat agents, including alkyl sulfonates, and anti-fog agents for use in plastic films. Furthermore, American Cyanamid Industrial Chemical Division markets "Aerosol™ OT Surface Active Agent" (dioctylsodium-sulfosuccinate), which is advertised as useful to prepare an anti-fog composition for direct application to glass.

None of the above-described coatings which reduce the tendency for an article to fog have anti-reflective properties. Furthermore, in general, the anti-fog compositions of the prior art rely on high solution concentrations (e.g., in excess of 0.2 percent, and typically in concentrations in excess of 5 percent by weight) of surfactant and other organic additives to provide an anti-fog effect. When used at such high concentrations, the surfactants and other organic additives would interfere with and significantly reduce the anti-reflective properties provided by porous coatings, such as metal oxides.

Face masks and shields which are described as having anti-fog and anti-glare properties are known. For example, the "SHIELDMATE" by IREMA U.S.A. Ltd. of Chicopee, M.A. is described in U.S. Patent No. 4,944,294 (Borek). The hospital face mask is described as including a transparent plastic eye shield coated with any suitable anti-fogging, anti-glare silicone agent, such as a dimethylsiloxane polymer.

World Patent Application No. 89/10106 (Russell) discloses a surgical mask/face shield combination. The face shield is coated with an anti-fog coating, such as that described in U.S. Patent No. 4,467,073. These coatings are made by combining, for example, polyvinylpyrrolidone, a surfactant, and a curable isocyanate functional prepolymer. Additionally, Infection Control Products, Inc., markets the "AGAFAR™ Adjustable Flip-Up Face Shield" which is advertised as being anti-glare, anti-fog and anti-reflective.
However, none of these products utilize a porous coating and none display an increase in transmission of visible light through the coated article of more than 2 to 3 percent greater than the uncoated article. It is understood that an increase in percent transmission corresponds to a decrease in percent reflection, provided the sample is transparent (i.e., non-light-absorbing and not hazy). Accordingly, a need exists for a coating composition which will impart anti-fog properties to a substrate coated therewith, while increasing the percent transmission, and correspondingly decreasing the percent reflection, of incident light through the substrate, such that the substrate is truly "anti-reflective."

The present invention provides coating compositions which impart both anti-reflection and anti-fog properties to substrates coated therewith. By "anti-reflective" it is meant that the percent transmission of a light transmissive substrate coated with the coating composition is increased by at least 3 percent over the uncoated substrate measured using 550 nm light. The coating composition utilizes an inorganic metal oxide in combination with particular anionic surfactants which are present in a concentration which imparts a durable anti-fog property to the coated substrate, yet does not destroy the anti-reflective properties provided by the metal oxide.

The present invention provides a coating composition comprising:
(a) an inorganic metal oxide sol capable of forming a porous inorganic metal oxide network which provides anti-reflective properties to a substrate;
(b) a surfactant having a solubility in water of less than 10 percent by weight at 23°C and comprised of at least one hydrophobic group and at least one covalently bonded hydrophilic anionic group, wherein
   (i) the hydrophilic anionic group comprises an anion selected from the group consisting of -OSO₂O⁻, -SO₂O⁻, -CO₂⁻, (-O)₂P(O)O⁻, -OP(O)(O⁻)₂, -P(O)(O⁻)₂, -P(O⁻)₂, -OP(O⁻)₂, (-SO₂)₂N⁻, -SO₂N(R)⁻, (-SO₂)₂C⁻H, and -N⁺ (R)₂(CH₂)ₓL', wherein R is hydrogen, an alkyl group which is unsubstituted or substituted with atoms independently selected from the group consisting of oxygen, nitrogen and sulfur, or an alkylene carboxyl group, which alkyl or alkylene carboxyl group comprises about 1 to 10 carbon atoms; x is 1 to 4; and L' is selected from the group consisting of -OSO₂O⁻, -SO₂O⁻, (-O)₂P(O)O⁻, -OP(O)(O⁻)₂, -P(O)(O⁻)₂ and -CO₂⁻; and wherein each anionic group is associated with or covalently bound to at least one cation, which cation is selected from the group consisting of H⁺, Na⁺, K⁺, Li⁺, Ca⁺², Mg⁺², Sr⁺², Al⁺³ , and R"A⁺, wherein R" is R or R' wherein R is hydrogen or an alkyi or cycloalkyl group of about 1 to 10 carbon atoms, and R' is covalently bonded to the surfactant molecule and is an alkyl bridging group of 1 to 10 carbon atoms, and A⁺ is N⁺R₃, a guanidinium ion optionally substituted with oxygen, nitrogen or sulfur atoms, or N⁺B wherein B comprises 3 to 7 atoms selected from the group consisting of carbon, nitrogen, sulfur and oxygen atoms which complete the nitrogen containing heterocyclic ring; and wherein any R or R' group may be unsubstituted or substituted with atoms independently selected from the group consisting of oxygen, nitrogen or sulphur; the cation being selected such that the net charge of the surfactant molecule is neutral; and
   (ii) wherein the hydrophobic group comprises a hydrocarbcn chain comprising at least 4 carbon atoms or a perfluorinated radical group comprising at least 3 carbon atoms,
   wherein the coating composition when coated on at least one side of a light transmissive substrate and dried provides a coated substrate with :
   (1) a porous inorganic metal oxide network of uniform average thickness;
   (2) a drop diameter of at least 4 mm when tested in accordance with the Wetting Test described herein; and
   (3) a percent transmission at 550 nm which is at least 3 percent greater than that of the uncoated substrate.

The compositions may optionally contain a coupling agent (e.g. a silane) and/or a polymeric binder that improves adhesion of the dried coating to the substrate.

Preferred coating compositions applied to at least one side of a light transmissive substrate increase the percent transmission of the substrate by at least 5 percent, and preferably by 10 percent, while resisting fogging even upon exposure to "steam," i.e., warm air saturated with water. The anti-fog property is shelf stable and deteriorates very slowly when exposed to accelerated aging conditions, as described hereinafter. Ideally, in preferred embodiments, the coated articles have exceptional anti-fog properties while also having greater than 96 percent transmission of 550nm light.

The compositions may be applied to a wide variety of substrates by a variety of coating methods. Accordingly, the invention provides protective eyewear, such as surgical masks and face shields, as well as ophthalmic lenses, windows and windshields which have anti-reflective and anti-fog properties.

The invention also relates to a method of imparting anti-reflection and anti-fogging properties to a substrate. The method comprises the steps of providing a substrate, preparing a coating composition having the formulation described above, applying the coating composition to the substrate, and drying the coating composition.

### Anti-reflection

The anti-reflective properties of the coatings of this invention are provided by a porous inorganic metal oxide network. More particularly, the coating compositions of the invention when coated on a substrate and dried provide a continuous and highly porous network of metal oxide particles. As used herein, the term "continuous" refers to a coating having no visible discontinuities or gaps. The term "network" (as used herein) refers to a porous, three-dimensional structure, preferably formed by an aggregation of colloidal particles linked together. The network is held together through particle/particle, particle/coupling agent or particle/coupling agent/particle bonds, providing a coating having integrity which does not flake off by simple flexing and/or use of the coated article.

The term "porous" refers to the presence of voids between the inorganic metal oxide particles created when the particles pack together. For single layer coatings, it is known that in order to maximize light transmission in air through an optically transparent substrate, and minimize reflection by the substrate, the refractive index of the coating should equal as closely as possible the square root of the refractive index of the substrate and the thickness of the coating should be one-fourth (¼) of the optical wavelength of the incident light. The voids in the coating provide a multiplicity of subwavelength interstices between the metal oxide particles where the index of refraction (IR) abruptly changes from that of air (IR=1) to that of the metal oxide particles (e.g., for silica IR=1.44). By adjusting the porosity, a coating having a calculated index of refraction (as shown in U.S. Patent No. 4,816,333 (Lange, et al.)) very close to the square root of the refractive index of the substrate can be created. By utilizing coatings having optimal indices of refraction, at coating thicknesses equal to approximately one-fourth the optical wavelength of the incident light, the percent transmission of light through the coated substrate is maximized and reflection is minimized.

The voids in the coating are present substantially throughout; however, the coating may vary in density, e.g., the coating may become gradually more porous moving away from the substrate producing a gradient density. Such a gradient density enhances the anti-reflective property of the coating. Preferably, the network has a porosity of about 25 to 45 volume percent, more preferably about 30 to 40 volume percent, when dried. Porosity may be calculated from the refractive index of the coating according to published procedures such as in W. L. Bragg, A. B. Pippard, Acta Crystallographica, volume 6, page 865 (1953). When the metal oxide is silicon dioxide, this porosity provides a coating having an index of refraction of 1.2 to 1.4, preferably 1.25 to 1.36, which is approximately equal to the square root of the refractive indices of polyester, polycarbonate, or polymethyl methacrylate substrates. For example, a porous silica coating having a refractive index of 1.25 to 1.36 is capable of providing a highly anti-reflective surface when coated on a polyethylene terephthalate substrate (IR=1.64) at a thickness of 1000-1200Å.

The metal oxide component of the present invention is preferably silica (essentially silicon dioxide with or without other additives or impurities) but may alternatively be aluminum oxide, tin oxide, titanium oxide, antimony oxide, zirconium oxide, as well as mixtures and combinations thereof. The metal oxide particles should be less than about 200nm in diameter in order to provide effective anti-reflective properties. Preferably the average particle diameter is less than 70nm, more preferably less than 20nm, and most preferably between about 4 and 8nm. Although the particles are preferably spherical, other shapes are possible including irregular and fibrous shapes. The metal oxide concentration is preferably from about 0.1 to 15 percent by weight of the coating solution, more preferably from about 0.5 to 5 percent by weight. Above about 15 percent by weight the coating solution becomes difficult to apply in the desired thickness range and below about 0.1 percent by weight, excessive time periods are required for the coating to dry after application to the substrate. The term "solution" as used herein includes dispersions or suspensions of finely divided inorganic metal oxide particles in a liquid medium.

The metal oxide is most conveniently coated on the substrate as a colloidal dispersion (referred to herein as a "sol") which comprises finely divided solid inorganic metal oxide particles in an aqueous or an organic liquid. The sol may be acid or base stabilized. Sodium hydroxide base stabilized sols having a pH of 9 to 11 are most preferred and include "NALCO 1115" and "NALCO 1130," commercially available from NALCO Chemical Co., "Remasol SP30," commercially available from Remet Corp., and "LUDOX SM," commercially available from E. I. Du Pont de Nemours Co., Inc.

### Anti-fog

The coating compositions of the present invention provide anti-fog properties, in addition to anti-reflection, to substrates coated therewith. Coatings are considered anti-fogging if a coated substrate resists the formation of small, condensed water droplets in sufficient density to significantly reduce the transparency of the coated substrate such that it cannot be adequately seen through, after exposure to repeated human breathing directly on the article and/or after holding the article above a "steam" jet. A coating composition may still be regarded as anti-fogging even though a uniform water film or a small number of large water droplets forms on the coated substrate so long as the transparency of the coated substrate is not significantly reduced such that it cannot be readily seen through. In many instances, a film of water that does not significantly reduce the transparency of the substrate will remain after the substrate has been exposed to a "steam" jet.

The compositions of the present invention derive their anti-fogging property by incorporation of a particular surfactant or combination of surfactants. The term "surfactant" as used herein describes molecules comprising hydrophilic (polar) and hydrophobic (non-polar) regions on the same molecule which are sizeable enough to be capable of reducing the surface tension of the coating solution and providing a coating which imparts anti-fog properties to substrates or articles coated therewith. Certain surfactants of the present invention comprise multiple hydrophilic and or hydrophobic regions on the same molecule.

Useful surfactants comprise at least one hydrophilic anionic group. The anionic group may be -OSO₂O⁻, -SO₂O⁻ , -CO₂⁻, (-O)₂P(O)O⁻, -OP(O)(O⁻)₂, -P(O)(O⁻)₂, -P(O⁻)₂, -OP(O⁻)₂, (-SO₂)₂N⁻, -SO₂N(R)⁻, (-SO₂)₂C⁻H or -N⁺(R)₂(CH₂)ₓL', wherein R is hydrogen, an alkyl group which is unsubstituted or substituted with atoms independently selected from the group consisting of oxygen, nitrogen and sulfur or an alkylene carboxyl group, which alkyl or alkylene carboxyl group comprises about 1 to 10 carbon atoms; x is 1 to 4; and L' is selected from the group consisting of -OSO₂O⁻ , -SO₂O⁻, (-O)₂P(O)O⁻, -OP(O)(O⁻)₂, -P(O)(O⁻)₂ and -CO₂⁻. Each anionic group is associated with at least one cation such that the ratio of total anionic charge of the surfactant molecule, to the total cationic charge of the surfactant molecule equals 1, making the net charge of the surfactant molecule neutral. The cation(s) are selected from the group consisting of hydrogen, sodium, potassium, lithium, ammonium, calcium, magnesium, aluminum, strontium and R"A⁺ groups, wherein R" is R or R', wherein R is hydrogen or an alkyl or cycloalkyl group of about 1 to 10 carbon atoms, and R' is covalently bonded to the surfactant molecule and is an alkyl bridging group of 1 to 10 carbon atoms, and A⁺ is N⁺R₃, a guanidinium ion optionally substituted with oxygen, nitrogen or sulfur atoms, or N⁺B wherein B comprises 3 to 7 atoms selected from the group consisting of carbon, nitrogen, sulfur and oxygen atoms which complete the nitrogen containing heterocyclic ring; and wherein any R or R' group may be unsubstituted or substituted with oxygen, nitrogen or sulfur atoms. Of course, cations having a charge greater than one may be associated with more than one anion, e.g., (SO₄)₂Ca or (SO₃)₂Mg. The anionic group may be the sole hydrophilic group or may be covalently bound to other hydrophilic groups such as ester, thio ester, ether, amide, urea, urethane, hydroxyl and amine groups and polymers comprising these groups and having molecular weights less than about 5,000, and preferably less than about 2,000 (e.g., an anionic derivative of a polyethoxylated surfactant).

Applicants have found that useful surfactants having a carboxylate group as the hydrophilic group further comprise an additional polar substituent capable of stabilizing the ionic form of the surfactant. Preferably, the additional polar substituent is no further than three or four atoms removed from the carbon of the carboxylate group. The added polar substituent is preferably an ether, amide, alcohol, carboxyl, ester, urea or urethane group.

The anionic property of the surfactants of the present invention is an important one. Applicants have found that surfactants such as the nonionic surfactants based on repeating units of ethylene oxide and propylene oxide (e.g., "Pluronic™ Block Copolymer Surfactants" and "Tetronic™ Block Copolymer Surfactants," both commercially available from BASF Corp., Performance Chemicals, Parsippany, New Jersey), as well as those based on tetramethyldecyne diol (e.g., "Surfynol 104," commercially available from Air Products and Chemicals, Inc., Allentown, Pennsylvania), do not produce a durable anti-fog coating when used with porous metal oxide networks. Additionally, polyethoxylated alcohols, such as "Tergitol™ TMN-6," commercially available from Union Carbide Chemical and Plastics Co., Industrial Chemicals Division, Danbury, Connecticut, polyethoxylated alkyl phenols, such as "TRITON™ X-100," also commercially available from Union Carbide, and amine oxides, such as "Rhodamox LO," commercially available from Rhone-Poulenc, Surfactant and Specialty Division, Dalton, Georgia, do not produce durable anti-fog coatings when used with porous metal oxide networks. Cationic surfactants such as 3-lauramidopropyltrimethyl-ammonium methosulfate (commercially available as "Cyastat™ LS Antistatic Agent", from Cytec Industries, Stamford, Conn.) and myristyl trimethylammonium bromide also do not produce durable anti-fog coatings when used with porous metal oxide networks.

Useful surfactants comprise at least one hydrophobic group which comprises a hydrocarbon chain comprising at least four carbon atoms, or a perfluorinated group comprising at least three carbon atoms. Surfactants containing a perfluorinated group preferably include a perfluorinated radical group of at least six carbons, more preferably at least eight carbon atoms. Surfactants which do not include a perfluorinated group preferably have a hydrocarbon chain of at least eight, and more preferably, at least twelve carbon atoms.

The surfactants of the present invention in order to be immobilized on the substrate at room temperature, preferably also possess at least one of the following characteristics.
1. The surfactant has a melting point greater than room temperature, i.e., greater than about 20°C, preferably greater than about 30°C, and most preferably greater than 40°C.
2. The surfactant is relatively insoluble in water. Preferably the surfactant has a solubility in water of less than about 1 percent by weight and more preferably less than about 0.1 percent by weight, at 23°C. Relatively insoluble surfactants are preferred since they are less likely to rehydrate, dissolve, and reorient, even under high humidity conditions.
3. The surfactant is capable of being covalently bound to metal oxide. The surfactant may itself react with the metal oxide, or may be chemically bound to the metal oxide through the use of a coupling agent, as described in further detail hereinbelow.

### Surfactant Chemistry

The surfactants useful in the practice of this invention have the following general structure:

[(R)ₐL^{-c}]_{d}(M^{+b})ₑ

wherein:
R is a perfluorinated alkyl or cycloalkyl group of about 3 to 18 carbon atoms; a polyethoxylated perfluoroalkyl or perfluorocycloalkyl substituted alcohol comprising about 3 to 18 perfluorinated carbon atoms and about 0 to 30 non-fluorinated carbon atoms; a perfluoroalkyl substituted alkyl or alkenyl group of about 3 to 18 perfluorinated atoms and about 0 to 30 non-fluorinated carbon atoms, which alkyl or alkenyl group optionally comprises oxygen, nitrogen or sulfur atoms within or substituted upon the alkyl or alkenyl chain; an alkyl or alkenyl group (straight or branched chain) of about 4 to 36 carbon atoms, which alkyl or alkenyl group optionally comprises oxygen, nitrogen or sulfur atoms within or substituted upon the alkyl or alkenyl chain; an aralkyl group of about 7 to 36 carbon atoms, which aralkyl group is optionally independently substituted in available positions by oxygen, nitrogen or sulfur atoms; or a polyethoxylated or polypropoxylated alkyl or aralkyl group which alkyl or aralkyl group comprises about 7 to 36 carbon atoms;
L is a sulfate (-OSO₂O⁻), sulfonate (-SO₂O⁻), phosphate ((-O)₂P(O)O⁻ or -OP(O)(O⁻)₂), phosphonate (-P(O)(O⁻)₂), sulfonimide ((-SO₂)₂N⁻), sulfonamide (-SO₂N(R')⁻), carboxylate (-CO₂⁻), phosphonite (-P(O⁻)₂), phosphite (-OP(O⁻)₂), or disulfonylmethide ((-SO₂)₂C⁻H). Amphoteric alkyl forms of the above groups are also useful, including groups having the formula -N⁺(R"')₂(CH₂)ₓL', wherein R"' is hydrogen or an alkyl or alkylene group optionally substituted with nitrogen, oxygen or sulfur atoms; or an alkylene carboxyl group, which alkyl or alkylene carboxyl group comprises about 1 to 10 carbon atoms; x = 1 to 4; and L' is -OSO₂O⁻, -SO₂O⁻, (-O)₂P(O)O⁻, -OP(O)(O⁻)₂, -P(O) (O⁻)2, -CO₂⁻, -P(O⁻)₂, or -OP(O⁻)₂; provided that when L is a carboxylate, R further comprises an additional polar heteroatom or substituent no further than four, and preferably no further than three, atoms removed from the carboxylate group wherein said polar substituent is an ether, amide, alcohol, carboxyl, ester, thioester, urea, or urethane group, or combinations thereof including oligomers comprising these polar groups;
M is hydrogen (H⁺), sodium (Na⁺), potassium (K⁺), lithium (Li⁺), ammonium (NH₄⁺), calcium (Ca⁺²), magnesium (Mg⁺²), strontium (Sr⁺²), aluminum (Al⁺³) or R"A⁺, wherein R" is R or R', wherein R is hydrogen or an alkyl or cycloalkyl group of about 1 to 10 carbon atoms and R' is covalently bonded to the surfactant molecule and is an alkyl bridging group of about 1 to 10 carbon atoms, and A⁺ is selected from the group consisting of N⁺R₃, (e.g., N⁺(CH₃R)₄, HN⁺(CH₂CH₂OH)₃, H₂N⁺(CH₂CH₂OH)₂); a guanidinium ion optionally substituted with oxygen, nitrogen or sulfur atoms; or a heterocyclic N⁺B wherein B comprises 3 to 7 atoms selected from the group consisting of carbon, nitrogen, sulfur and oxygen atoms which complete the nitrogen containing heterocyclic ring; and wherein R' and R may be substituted in available positions with oxygen, nitrogen or sulfur atoms;
   a and c are independently 1 or 2;
   b and d are independently 1, 2 or 3; and
   e is equal to (c times d)/b, or O in the case of amphoteric surfactants.

When R is a polyethoxylated or polypropoxylated substituent or a copolymer of ethylene oxide and propylene oxide, wherein these polymeric subunits are present in amounts of 1 to 100 moles, preferably about 1 to 20 moles per mole of surfactant.

The following surfactant classes and surfactants are particularly useful individually or in combination in the practice of the present invention.

### 1. Perfluoroaliphatic anionic salts

Surfactants within this class are of the general formula described above wherein:

R = CF₃CₙF₂ₙ-,

and wherein n is about 2 to 17, preferably about 3 to 12.

Preferred surfactants within this class include the lithium, sodium and potassium salts of anionic perfluoroaliphatic radical containing compounds. Some particularly preferred lithium salts include the following:

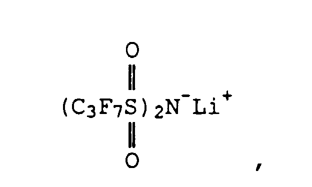

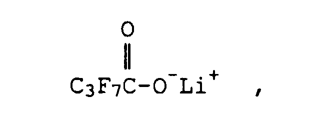

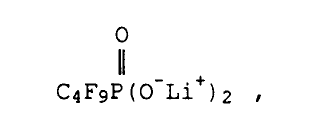

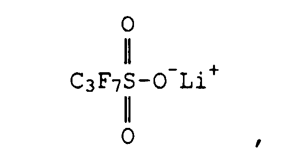

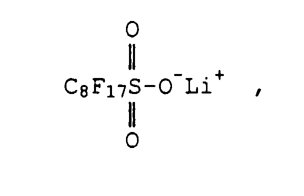

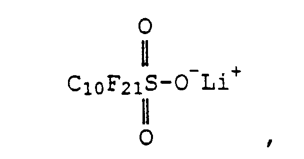

(C₃F₇SO₂)₂HC⁻Li⁺

Useful lithium salts are made by following techniques disclosed in, for example, U.S. Pat. No. 2,732,398 (Brice et al.) and U.S. Pat. No. 2,809,990 (Brown). Examples of commercially available lithium salts of anionic perfluoroaliphatic radical containing compounds include "Fluorad™ FC-122," "Fluorad™ FC-123" and "Fluorad™ FC-124 Fluorochemical Surfactants," from 3M Company, St. Paul, Minnesota.

Preferred potassium salts include: 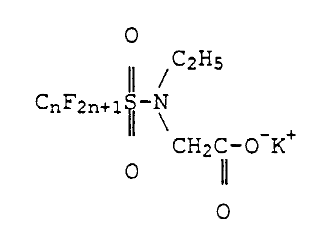 and CₙF₂ₙ₊₁SO₃⁻K⁺, wherein n is about 3 to 18, as well as mixtures of these salts.

Useful potassium salts are made by following techniques disclosed in, for example, U.S. Pat. No. 2,809,990 (Brown). Examples of commercially available potassium salts include "Fluorad™ FC-127," "Fluorad™ FC-129" and "Fluorad™ FC-95 Fluorochemical Surfactant," from 3M. A useful ammonium salt is commercially available as "Fluorad™ FC-120 Fluorochemical Surfactant" from 3M.

### 2. Perfluorinated radical substituted aliphatic anionic salts

Surfactants within this class are of the general formula described above, wherein:

R = (CₙF₂ₙ₊₁)_{z} R²-

and wherein:
n is about 2 to 36, preferably 6 to 12;
R² is a branched or straight chain alkylene or aralkylene of about 2 to 36 carbon atoms, preferably 2 to 22 carbon atoms, optionally independently substituted in available positions with oxygen, nitrogen or sulfur atoms, which R² group is selected such that R comprises at least 7 carbon atoms; and
z is about 1 to 3, preferably about 1 or 2.

Examples of commercially available salts of this class include "Zonyl™ FSA Fluorosurfactant"
(F(CF₂CF₂)₃₋₈CH₂CH₂SCH₂CH₂CO₂⁻Li⁺) and "Zonyl™ FSE
Fluorosurfactant" (a mixture of
F(CF₂CF₂)₃₋₈CH₂CH₂OP(O)(O⁻NH₄⁺)₂ and
[F(CF₂CF₂)₃₋₈CH₂CH₂O]₂P(O)(O⁻NH₄⁺), from E.I. Du Pont de Nemours and Co.

### 3. Straight or branched chain aliphatic sulfates and sulfonates

Surfactants within this class are of the general formula described above, wherein:

R = CₙH₂ₙ₊₁(R²)ₘ-

and wherein:
n is about 4 to 36,
R² is a branched or straight chain alkyl or aralkyl of about 1 to 36 carbon atoms, preferably 1 to 22 carbon atoms, optionally independently substituted in available positions with oxygen, nitrogen or sulfur atoms;
m is 0 or 1, and
L is SO₃⁻ or SO₄⁻.

Examples of commercially available surfactants of this class include sodium dodecyl sulfate and sulfonates such as "Mackam™ CS" 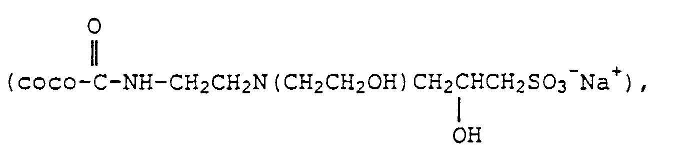 where "coco" means a mixture of alkyl chain lengths derived from coconut oil fatty acid residues, "Mackam™ CBS-50 Amphoteric" from The McIntyre Group Ltd., and "Hostastat HS-1" (C₁₀₋₁₈H₂₁₋₃₉SO₃⁻Na⁺) , from Hoechst Celanese Corp.

### 4. Sulfates or Sulfonates of polyethoxylated derivatives of straight or branched chain aliphatic alcohols and carboxylic acids

Surfactants within this class are of the general formula described above, wherein:

R = CₙH₂ₙ₊₁(CO)ₚO(CH₂CH₂O)_{y}CH₂CH₂-,

and wherein:
n is about 4-36,
p is 0 or 1 and
y is about 1-100, preferably 1-20; and
wherein L is SO₄⁻ or SO₃⁻.

Examples of commercially available surfactants of this class include "Steol CA-460" (C₁₂H₂₅O(CH₂CH₂O)₁₂SO₃⁻Na⁺), from Stepan Co.

### 5. Alkylbenzene or alkylnaphthalene sulfonates and sulfates

Surfactants within this class are of the general formula described above, wherein:

R = (CₙH₂ₙ₊₁)_{q}C₆H_{5-q}-

or

(CₙH₂ₙ₊₁)_{q}C₁₀H_{7-q}-

and wherein:
n is about 4 to 36, preferably 8 to 22,
q is 1-3, preferably 1 or 2, and
L is SO₃⁻ or SO₄⁻.

Examples of commercially available surfactants of this class include "Rhodocal™ DS-10" (sodium laurylbenzene sulfonate) from Rhone-Poulenc Co., "Polystep™ A-16" (C₁₂H₂₃-C₆H₆-SO₃⁻Na⁺) and "Polystep™ A-15," from Stepan Co., and "Poly-Tergent™ 2EP" from Olin Corp.

### 6. Ethoxylated and polyethoxylated alkyl and aralkyl alcohol carboxylates

Surfactants within this class are of the general formula described above, wherein:

R = (CₙH₂ₙ₊₁)_{q}(C₆H_{5-q})ₘO(CH₂CH₂O)_{y}CH₂-,

and wherein:
n is about 4 to 36, preferably 8 to 22,
m is 0 or 1, and
q is 1 or 2, preferably 1, and
y is about 1 to 100, preferably 1-20; and
wherein
L is CO₂⁻.

Examples of commercially available surfactants of this class include "Sandopan LS-24 Carboxylated Surfactant" (C₁₂H₂₅O(CH₂CH₂O)₁₂CH₂COO⁻Na⁺), "Sandopan L8-HC Carboxylated Surfactant" and "Sandopan LA-8 Carboxylated Surfactant" (C₁₂H₂₅O(CH₂CH₂O)₄CH₂COO⁻ Na⁺), from Sandoz Chemicals, Corp.

### 7. Glycinates

Surfactants within this class are of the general formula described above, wherein:

R = R²-C(O)N(R³)CH₂-,

wherein:
R² is a branched or straight chain alkyl of about 4 to 36 carbon atoms, preferably 8 to 22 carbon atoms, or an aralkyl group of about 7 to 36 carbon atoms, preferably 12 to 22 carbon atoms, which alkyl or aralkyl group is optionally independently substituted in available positions with oxygen, nitrogen or sulfur atoms; and
R³ is hydrogen or an alkyl group of about 1 to 10 carbon atoms which may be optionally independently substituted in available positions by oxygen, nitrogen or sulfur atoms;
and wherein L is CO₂⁻.

Examples of preferred surfactants within this class are alkyl sarcosinates and alkyl glycinates. Examples of commercially available surfactants of this class include "Hampshire™ C-30," (coco-C(O)N(CH₃)CH₂COO⁻Na⁺) from Hampshire™ Chemical Co., and "Mackam™ Amphoteric" (dihydroxyethyl tallow glycinate) from the McIntyre Group, Ltd.

### 8. Sulfosuccinates

Surfactants within this class are of the general formula described above, wherein: 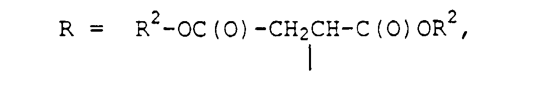 and wherein:
R² is a branched or straight chain alkyl group of about 4 to 36 carbon atoms, preferably 8 to 22 carbon atoms, or an aralkyl group of about 7 to 36 carbon atoms, preferably 12 to 22 carbon atoms, which alkyl or aralkyl group may be independently substituted in available positions by oxygen, nitrogen and/or sulfur atoms; and
L is SO₃⁻.

An example of a preferred surfactant of this class is dialkyl sulfosuccinate. Examples of commercially available surfactants of this class include "Aerosol™ OT Surface Active Agent" (C₈H₁₇OC(O)-CH(SO₃⁻Na⁺)CH₂C(O)O-C₈H₁₇) and "Aerosol™ TR Surface Active Agent" (C₁₃H₂₇-OC(O)-CH(SO₃⁻Na⁺)CH₂C(O)O-C₁₃H₂₇) from Cytec Industries.

### 9. Isethionate Derivatives

Surfactants within this class are of the general formula described above, wherein:

R = R²-C(O)OCH₂CH₂-

and wherein
R² is a branched or straight chain alkyl group of about 4 to 36 carbon atoms, preferably 8 to 22 carbon atoms, or an aralkyl group of about 7 to 36 carbon atoms, preferably 12 to 22 carbon atoms, which alkyl or aralkyl group is optionally independently substituted in available positions with oxygen, nitrogen or sulfur atoms; and
L is SO₃⁻.

Examples of commercially available surfactants of this class include "Igepon™ AC-78" (coconut acid ester of sodium isethionate), from GAF Corp., New York, New York.

### 10. N-acyltaurine Derivatives

Surfactants within this class are of the general formula described above wherein:

R = R²-C(O)N(R³)CH₂CH₂-

and wherein
R² is a branched or straight chain alkyl group of about 4 to 36 carbon atoms, preferably 8 to 22 carbon atoms, or an aralkyl group of about 7 to 36 carbon atoms, preferably 12 to 22 carbon atoms, which alkyl or aralkyl group is optionally independently substituted in available positions with oxygen, nitrogen or sulfur atoms;
R³ is hydrogen or an alkyl group of about 1 to 10 carbon atoms which may be optionally independently substituted in available positions by oxygen, nitrogen or sulfur atoms; and
L = SO₃⁻.

Examples of commercially available surfactants of this class include "Igepon™ T-77" (sodium N-methyl-N-oleyltaurate), from GAF Corp.

### 11. Amphoteric Alkyl Carboxylates

Surfactants within this class are of the general formula described above, wherein: 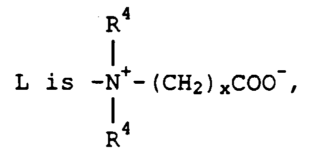 wherein R⁴ is hydrogen, or an alkyl or alkylene carboxyl group of about 1 to 8 carbon atoms optionally substituted in available positions by nitrogen, oxygen or sulfur atoms, and x is 1 to 4; and
wherein R is a branched or straight chain alkyl group of about 4 to 36 carbon atoms or an aralkyl group of about 7 to 36 carbon atoms which alkyl or aralkyl group is unsubstituted or independently substituted in available positions with oxygen, nitrogen or sulfur atoms.

Examples of preferred surfactants of this class are amphoteric propionates and alkyl and aryl betaines, optionally substituted with oxygen, nitrogen and/or sulfur atoms. Examples of commercially available surfactants of this class include "Tego™ Betain F-50" (coco-C(O)NH-CH₂CH₂CH_{2⁻}N⁺(CH₃)₂-CH₂COO⁻), from Goldschmidt Chemical Corp., "Mackam™OB-30 Amphoteric" (C₁₈H₃₄N⁺(CH₃)₂CH₂COO⁻), "Mackam™ HV Amphoteric" (C₁₈H₃₄C(O)NHCH₂CH₂CH₂N⁺(CH₃)₂CH₂COO⁻) from the McIntyre Group, Ltd., "Miranol 2CIB" from Rhone-Poulenc, Co., and "Miratane™ AP-C" (coco₂-N⁺H-CH₂CH₂COO⁻) from Rhone-Poulenc Co.

### 12. Alkyl phosphate mono or di-esters

Surfactants within this class are of the general formula described above, wherein:

R = R²O(CH₂CH₂O)ᵥCH₂CH_{2⁻} ,

and wherein
R² is a branched or straight chain alkyl group of about 4 to 36 carbon atoms, preferably 8 to 22 carbon atoms, or an aralkyl group of about 7 to 36 carbon atoms, preferably 12 to 22 carbon atoms, optionally independently substituted in available positions with oxygen, nitrogen or sulfur atoms;
v is 0-100, preferably 0-20; and
L is PO₄⁻² or PO₄⁻.

Examples of commercially available surfactants of this class include "Rhodafac™ MC-470" (ethoxylated dodecyl alcohol phosphate ester, sodium salt) from Rhone-Poulenc, and "Sipostat 0012" (C₁₂H₂₅OP(O)(O⁻Na⁺)₂) and "Sipostat 0018" (C₁₈H₃₇OP(O)(O⁻Na⁺)₂) from Specialty Industrial Products, Inc., Spartanburg, South Carolina.

Applicants have discovered that the surfactants of the present invention can be utilized in concentrations which are effective to provide the coating composition with anti-fog properties, yet will not destroy the anti-reflective effects produced by the inorganic metal oxide. The anti-reflective property of the coating may be decreased by the surfactant, or by other additives, by one or both of two means. First, if too much surfactant is added, the void volume of the coating decreases thereby increasing the refractive index of the coating beyond that desired for maximum transmission of light. Secondly, the refractive index of the surfactant or additive can itself influence the refractive index of the coating. In general, the highest concentration of surfactant which will not adversely effect the anti-reflective property of the coating composition or the coating quality is preferred. Surfactants of lower refractive indices may be tolerated at higher concentrations on a weight basis. Rinsing or steeping the coated article in water may be desirable to remove excess surfactant or other additive. For typical concentrations of metal oxide (e.g., about 1 to 5 percent by weight) most surfactants comprise less than about 0.15 percent by weight of the coating composition, preferably less than 0.10 percent by weight, more preferably between about 0.003 and 0.05 percent by weight, and most preferably between about 0.01 and 0.05 percent by weight, in order to preserve the anti-reflective properties of the coating. It should be noted that with some surfactants a spotty coating is attained at concentrations in excess of what is needed to achieve the anti-fog property.

The surfactant may be applied as part of the metal oxide coating composition or may be applied, preferably in an aqueous or hydroalcoholic medium, as an "overcoat", i.e., can be applied as a separate coating solution over a previously deposited metal oxide coating. Preferably, the surfactant is added directly to the metal oxide sol coating composition to simplify the coating process and to minimize any risk of scratching the metal oxide layer.

### Other Additives

Many of the surfactants of the present invention not only impart anti-fog properties to the film but also lower the surface tension of aqueous coating solutions such that the solution uniformly wets and coats the article. In some instances, however, in order to ensure uniform coating of the article from an aqueous or hydroalcoholic solution it may be beneficial to add a wetting agent, which is typically a surfactant, including many of the surfactants described herein, as well as surfactants that do not impart durable anti-fog properties. Examples of useful wetting agents include polyethoxylated alkyl alcohols (e.g. "Brij 30," and "Brij 35," commercially available from ICI Americas, Inc., and "Tergitol™ TMN-6™ Specialty Surfactant," commercially available from Union Carbide Chemical and Plastics Co.), polyethoxylated alkylphenols (e.g., "Triton™ X-100" from Union Carbide Chemical and Plastics Co., "Iconol NP-70" from BASF Corp.) and polyethylene glycol/polypropylene glycol block copolymer (commercially available as "Tetronic™ 1502 Block Copolymer Surfactant," "Tetronic™ 908 Block Copolymer Surfactant" and "Pluronic™ F38 Block Copolymer Surfactant," all from BASF, Corp.) Of course, any added wetting agent must be included at a level which will not destroy the anti-reflective or anti-fog properties of the coating. Generally the wetting agent is used in amounts of up to about 0.10 weight percent of the coating composition depending on the amount of inorganic metal oxide. Preferably the wetting agent is present in amounts less than 0.05, more preferably less than 0.03, weight percent of the coating composition. Alternatively, lower alcohols (C₁ to C₈) in the coating solution have proven useful in improving wetting.

The coating compositions of the present invention may also include a coupling agent capable of covalently bonding the surfactant to the metal oxide. Some coupling agents are capable of reacting with specific functional groups on the surface of the article to be coated. Consequently, the coupling agent may be capable of promoting adhesion of the coating composition to the substrate. The coupling agent has at least two reactive functionalities. One reactive functionality is capable of covalently bonding to the metal oxide and the second is capable of covalently bonding to the surfactant. For example, reactive functionalities such as amino, hydroxyl, mercaptan, acrylate and methacrylate groups present on one compound (the surfactant, coupling agent, or the metal oxide) can react with complementary reactive functionalities, such as oxirane, chloro-, bromo-, iodo-, alkyl, aziridine, anhydride, acrylate, methacrylate, or isocyanato groups, present on the other compound (coupling agent or surfactant). More than one coupling agent may be used. For example, two types of coupling agents which are capable of covalently bonding to each other may be employed where one coupling agent is capable of covalently bonding to the metal oxide and the other is capable of covalently bonding to the surfactant.

Useful silane coupling agents include those with the following formula: 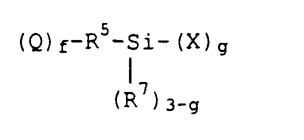 wherein:
R⁵ is a substituted or unsubstituted divalent hydrocarbon bridging group of about 1 to 20 carbon atoms, optionally including in the backbone 1 to 5 moieties selected from the group consisting of -O-, -C(O)-, -S-, -SO₂- and -NR⁶- groups, and optionally substituted on the backbone by -OH, -SH, or -NR⁶-₂ wherein R⁶ is hydrogen, acetyl, or a hydrocarbon group of 1 to 6 carbon atoms;
X is -OR⁸ where R⁸ is an alkyl, aryl, heteroaryl, or aralkyl group of 1 to 8 carbon atoms, preferably methyl or ethyl; or -N=C(R⁹)₂, wherein R⁹ is independently an alkyl, aryl or aralkyl group of 1 to 8 carbon atoms;
R⁷ is independently an alkyl, aryl, aralkyl or alkoxy group of 1 to 8 carbon atoms optionally substituted in available positions by oxygen, nitrogen and/or sulfur atoms;
f is 0, 1, or 2;
g is 2 or 3; and
Q is a reactive functional group capable of reacting with complementary functionalities on the surface of the substrate or the surfactant. Examples of Q include amino; hydroxyl; mercaptan; oxirane; chloro-, iodo-, and bromo-alkyl; aziridine; cyclic carboxylic anhydride; acrylate; methacrylate; acrylamide, azide, and isocyanato groups. It should be understood that when present in the coating compositions of the invention (particularly with base stabilized sols) the coupling agents will hydrolyze, in which case one or more of the "X" or "OR²" groups will be converted to a silanol or silanolate.

Preferred silanes have the structure:

(Q)_{f}-R⁵-Si(OR⁸)₃,

wherein Q is preferably a primary or secondary epoxy or amino group and R⁵ and R⁸ are as described above.

Additional information on ambifunctional silane coupling agents may be found in European Patent Application No. 0,372,756 A2. Alternatively the coupling agent can be a titanate or zirconate compound, such as "Tyzor™ Titanate," commercially available from Du Pont.

The amount of coupling agent included in the coating composition should be limited in order to prevent destruction of the anti-reflective or anti-fog properties of the coating. The optimal amount of coupling agent is easily determined experimentally and is a function of the coupling agent's identity, molecular weight and refractive index. The coupling agent(s), when present, are typically added to the composition at levels of 0.1 to 20 percent by weight of the metal oxide concentration, and more preferably about 1 to 10 percent by weight of the metal oxide. Tetraalkoxy coupling agents, such as tetraethylorthosilicate (TEOS) and oligomeric forms such as alkyl silicates (e.g. poly(diethoxy siloxane)), may also be useful to improve binding between metal oxide particles.

Additional materials capable of bonding with the metal oxide and improving the durability of the anti-fog coatings of this invention include silane agents. Preferred silane agents are particular anionic silanes (described below) which are capable of themselves providing anti-fog properties to substrates or articles coated therewith. Such preferred anionic silanes are described in commonly assigned copending United States patent application, Attorney's Docket No. 51197USA8A, filed upon the same date as this application. The term anionic silane as used herein describes organofunctional silicon containing compounds capable of hydrolyzing to organosilanol with subsequent condensation to organofunctional siloxane oligomers.

The preferred anionic silane compounds useful in the solutions and compositions of the present invention have the following general structure: 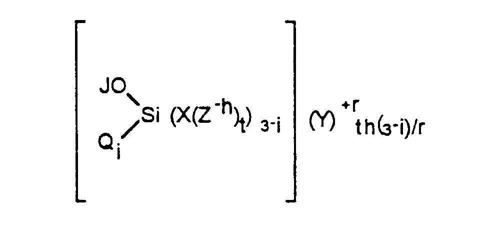 wherein:
Q is selected from the group consisting of hydroxyl, alkyl groups containing from 1 to about 4 carbon atoms, and alkoxy groups containing from 1 to about 4 carbon atoms;
J is selected from cations derived from the group consisting of hydrogen, alkali metals and organic cations of strong bases having an average molecular weight of less than about 150 and a pKa of greater than about 11;
X is an organic linking group;
Z is selected from the group consisting of -OSO₂O⁻, -SO₂O⁻, -CO₂⁻, (-O)₂P(O)O⁻, -P(O)(O⁻)₂, -OP(O) (O⁻)₂, -P(O⁻)₂ and -OP(O⁻)₂;
Y is selected from cations derived from the group consisting of hydrogen, alkali metals, alkali earth metals, organic cations of weak bases having an average molecular weight of less than about 200 and a pKa of about 8 to 11 (e.g., HN⁺(CH₂CH₂CH₂OH)₃ and H₂N⁺(CH₂CH₂OH)₂), organic cations of strong bases having an average molecular weight of less than about 150 and a pKa of greater than about 11, substituted and unsubstituted guanidines, and quaternary ammonium cations (e.g. N⁺(CH₃)₄, N⁺(CH₂CH₃)₄ and N⁺H₄); provided that J is hydrogen when Y is selected from cations derived from hydrogen, alkaline earth metals and said weak organic bases;
r is equal to the valence of Y and is 1 to 3;
h is 1 or 2;
i is 1 or 2; and
t is 1 to 3.

Preferably Z is sulfonate (SO₂O⁻) or phosphonate (-P(O)(O⁻)₂) or carboxylate (CO₂⁻), more preferably sulfonate and phosphonate, and the preferred anionic silane is an organosilanol, such as the sulfonato-organosilanols disclosed in U.S. Patent 4,235,638 to Beck. Alternatively, the anionic silane may be one of those disclosed in U.S. Patent Nos. 3,816,184; 4,344,860; or 4,370,255. The organic linking group X, is preferably selected from alkylene groups, cycloalkylene groups, hydroxy-substituted alkylene groups, hydroxy-substituted mono-oxa alkylene groups, divalent hydrocarbon groups having mono-oxa backbone substitution, divalent hydrocarbon groups having monothia backbone substitution, divalent hydrocarbon groups having monooxa-thia backbone substitution, divalent hydrocarbon groups having dioxa-thia backbone substitution, arylene groups, arylalkylene groups, and alkylarylene groups, all of which groups may be substituted by N, O and/or S atoms and all of which X groups comprise from about 1 to 20 carbon atoms, preferably from about 1 to 6 carbon atoms. Most preferably X is selected from alkylene groups, hydroxy-substituted alkylene groups and hydroxy-substituted mono-oxa alkylene groups.

In order to ensure optimum hydrophilicity and maximize the durability of the coating, the preferred anionic organosilanol preferably has a relatively high percentage of oxygen on a weight percentage basis. Preferably, the weight percent oxygen is at least about 30%, more preferably at least about 40%, and most preferably in the range of about 45 to 55%. In general, the weight percent silicon in these compounds is no greater than about 15%. Each of these percentages is based on the weight of the compound in the water-free acid form. Aqueous or hydroalcoholic solutions of the organosilanol-sulfonic acids (i.e. Z is SO₃⁻ and Y is hydrogen) are acidic generally having a pH of less than about 5 while the organo-silanolate-sulfonate salts are basic and generally have a pH of greater than about 9.0. In order to prevent destabilization of the preferred base stabilized metal oxide sols the organo-silanolate-sulfonate salt form is preferred.

The anionic organosilanol may be applied as part of the metal oxide and/or surfactant containing coating composition or may be applied as an "overcoat", i.e. can be applied as a separate coating solution applied over a previously deposited metal oxide and/or surfactant containing coating. Preferably, the anionic organo-silanol is added directly to the metal oxide sol and surfactant-containing coating composition to simplify the coating process and to minimize any risk of scratching the metal oxide layer.

The preferred anionic organosilanols are most conveniently applied from an aqueous or hydroalcoholic solution and therefore may be partially or completely hydrolyzed to the silanol/silanolate form and may include oligomeric siloxane forms of the anionic organosilanol. The level of organosilanol must be kept relatively low with respect to the metal oxide concentration in order to prevent reduction in the anti-reflective property. The anti-reflective property may be decreased by one or both of two means. Firstly, if too much organosilanol is added the porosity (void volume) of the coating decreases, thereby increasing the refractive index of the coating beyond that desired for maximum transmission of light. Secondly the refractive index of the silane itself might influence the refractive index of the coating if the amount of silane becomes excessive. In general, the highest level of anionic silane which will not adversely affect the anti-reflective property or coating quality is preferred. The anionic silanes are preferably added to the coating composition at a concentration of about 5 to 50% by weight of the metal oxide. More preferably the anionic silanes are added to the coating composition at a concentration of about 10 to 30% by weight of the metal oxide, in order to preserve the anti-reflective properties of the coating.

The coating composition may optionally contain a polymeric binder to improve scratch resistance and/or adhesion of the coating composition to the substrate. Useful polymeric binders are preferably water soluble or water swellable and include polymers comprised of ethenically unsaturated monomer(s), such as polyvinyl alcohol, polyvinylpyrrolidone, polyvinyl acetate, polyacrylates and methacrylates and polyurethanes; polyesters; natural polymers such as starch, gelatin, gums, celluloses, dextran, proteins and the like; and derivatives (ionic and non-ionic) and copolymers based on any of the polymers listed above. Furthermore, polymers comprising alkoxysilane functionalities may also be useful. The coating composition can contain up to about 5 weight percent of the polymeric binder based on the weight of the inorganic metal oxide. Useful amounts of polymeric binder are generally in the range of about 0.05 to 5 weight percent to improve scratch resistance and coating adhesion.

It is also possible to apply a primer coating to improve adhesion of the coating to the substrate. A particularly preferred primer material is polyvinylidene chloride (PVDC).

### Articles

Substrates to which the coating compositions of the invention can be applied are preferably transparent or translucent to visible light. Preferred substrates are made of polyester (e.g., polyethylene terephthalate, polybutyleneterephthalate), polycarbonate, allyldiglycolcarbonate, polyacrylates, such as polymethylmethacrylate, polystyrene, polysulfone, polyethersulfone, cellulose acetate butyrate, glass and the like, including blends and laminates thereof. Typically the substrate is in the form of a film, sheet, panel or pane of material and is part of an article such as ophthalmic lenses, architectural glazings, decorative glass frames, motor vehicle windows and windshields, and protective eye wear, such as surgical masks and face shields. The coatings may if desired, cover only a portion of the article, e.g., only the section immediately adjacent the eyes in a face shield may be coated. The substrate may be flat, curved or shaped. The article to be coated may be produced by blowing, casting, extrusion, or injection molding.

Articles such as disposable surgical face masks and face shields which are coated with the anti-reflective, anti-fog compositions of this invention are preferably stored in single use packages which reduce environmental exposure and contamination which can result in decreased anti-fog properties. Reusable articles are preferably used in combination with a package that protects or completely seals the product from environmental exposure when not in use. The material used to form the packages should be comprised of a non-contaminating material. It has been found that certain materials can result in partial or total elimination of the anti-fog properties. While not being bound by any theory, it is currently believed that materials which contain plasticizers, catalysts, and other low molecular weight materials which can volatilize on aging are sorbed into the coating and result in a decrease in the anti-fog property. For example, packaging materials such as polyurethane foams, plasticized polyvinylchloride and low density polyethylene have been found to significantly reduce the anti-fog properties of the articles of the present invention, especially when in direct contact with the coating. Currently preferred packaging materials include paper and bleached paper products, such as bleached white bond paper, cardboard, and clay-coated solid white bleached sulfate boxboard, and/or films or laminates made from polyester, high density polyethylene, or polystyrene.

### Process

The compositions of the present invention are preferably coated on the article using conventional techniques, such as bar, roll, curtain, rotogravure, spray, or dip coating techniques. The preferred methods include bar and roll coating or air knife coating to adjust thickness. In order to ensure uniform coating and wetting of the film, it is convenient to oxidize the substrate surface prior to coating using corona discharge or flame treatment methods. These methods may also improve adhesion of the coating to the substrate. Other methods capable of increasing the surface energy of the article include the use of primers such as thin coatings of polyvinylidene chloride (PVDC). The coatings of the present invention are preferably applied in uniform average thicknesses varying by less than about 200A, and more preferably by less than 100Å, in order to avoid visible color variations in the coating. The optimal average dry coating thickness is dependent upon the particular coating composition, but in general the average thickness of the coating is between 500 and 2500Å, preferably 750 to 2000Å, and more preferably 1000 to 1500Å, as measured using an ellipsometer such as a Gaertner Scientific Corp. Model No. L115C. Above and below this range, the anti-reflective properties of the coating may be significantly diminished. It should be noted, however, that while the average coating thickness is uniform, the actual coating thickness can vary considerably from one particular point on the coating to another. This variation in thickness, when correlated over a visibly distinct region, may actually be beneficial by contributing to the broad band anti-reflective properties of the coating.

In a preferred embodiment anti-reflection and anti-fogging properties are imparted to a substrate by
(b) preparing a first coating composition comprising an inorganic metal oxide sol capable of forming a porous inorganic metal oxide network which provides anti-reflective properties to a substrate;
(c) preparing a second coating composition comprising the aforementioned surfactant;
(d) applying either the first or second coating composition to at least one side of the substrate;
(e) allowing the coating applied in step (d) to dry;
(f) applying the coating composition not applied in step (d) to at least one side of the substrate;
(g) allowing the coating applied in step (f) to dry.

The coatings of the present invention are preferably coated on both sides of the substrate. Alternatively, the coatings of the present invention may be coated on one side of the substrate. The opposite side of the substrate may be:
a. uncoated,
b. coated with a conventional surfactant or polymeric anti-fogging composition such as that disclosed in US Patent Nos. 2,803,552; 3,075,228; 3,819,522; 4,467,073; or 4,944,294, or
c. coated with an anti-reflective composition, such as that disclosed in US Patent No. 4,816,333, or the multiple layered coating described by J.D. Masso in "Evaluation of Scratch Resistant and Anti-reflective Coatings for Plastic Lenses," (supra). Preferably, the coating surface should face the direction of higher humidity, e.g., on a face shield the side having the anti-fog coating should face the wearer.

Once coated, the article is typically dried at temperatures of between 20 and 150°C in a recirculating oven. The temperature may be increased further to speed the drying process, but care must be exercised to avoid degradation of the substrate. The preferred coating compositions are preferably dried at between 50 and 120°C and most preferably between 100 and 110°C. After the coating is applied to the substrate and dried, it comprises preferably from about 85 to 99.7 percent by weight (more preferably from about 88 to 95 percent by weight) metal oxide, about 0.25 to 5 percent by weight (more preferably from about 0.2 to 2 percent by weight) surfactant, up to about 25 percent by weight (more preferably from about 5 to 15 percent by weight) coupling agent and up to about 5 percent by weight (preferably up to about 2 percent by weight) wetting agent.

When the coating compositions of the invention are applied to substrates to provide anti-reflection properties, glare is reduced by increasing the light transmission of the coated substrate. Preferably, the coated substrate exhibits an increase in transmission of light of at least 3 percentage points and up to as much as 10 percentage points or more, when compared to an uncoated substrate, at 550nm (i.e., the wavelength at which the human eye displays peak photo-optic response). The percent transmission is dependent upon the angle of incidence and the wavelength of light and is determined using ASTM test method D1003-92, entitled "Haze and Luminous Transmittance of Transparent Plastics." Preferably, the coated substrates display an increase in percent transmission of greater than 3 percent, more preferably greater than 5 percent, and most preferably greater than 8 percent when compared with an uncoated substrate, using 550 nm light. When the desired usage involves significant "off-axis" (i.e., non-normal) viewing or unwanted reflections, gains in visibility may be greater especially where the reflections approach or exceed in brightness the object in view.

The coating compositions of the invention, as discussed hereinabove, provide anti-fog as well as anti-reflective properties to surfaces coated therewith. The anti-fog property is demonstrated by the tendency of the coatings to resist the formation of water droplets which tend to significantly reduce the clarity or transparency of the coated substrate. Water vapor from, for example, human breathing, tends to condense upon the coated substrate in the form of a thin uniform water film, rather than as water droplets. Such a uniform film does not significantly reduce the transparency of the substrate. For example, using the "Wetting Test" described in the Examples, when a 3 microliter drop of water is placed on the surface of a substrate coated with the coating composition of the invention, the drop spreads to an initial diameter of at least 6mm, preferably at least 7mm, and most preferably at least 8mm.

The coating compositions of the present invention are durable and shelf stable, e.g., they do not deteriorate significantly when allowed to be exposed at 23°C and 50% relative humidity for up to sixteen weeks. Preferred coatings when exposed at 30°C and 60% relative humidity in a recirculated environmental chamber (the circulation rate = 1.67 vol./min.) for at least fourteen days, and, more preferably, at least twenty-one days, and most preferably twenty-eight days, and tested according to the "Wetting Test" described in the examples, have a 3 microliter drop diameter of at least 4mm, and more preferably at least 5mm.

### Examples

The surfactants present in the anti-fog/anti-reflective compositions of Examples 1 through 63 and Comparative Examples A through AH are described in Table 1.

**TABLE 1**

| Ex. No. | Surfactant Class | Type - chemical description | Trade Name | Source/Address |
|---|---|---|---|---|
| 1, 20, 42 | Perfluorinated radical substituted aliphatic anionic salts | anionic - fluorochemical surfactant | Zonyl™ FSA Fluoro-Surfact ant | E. I. Du Pont de Nemours & Co., Chemicals Dept., Wilmington, DE |
| 2,19,41 | Sulfosuccinates | anionic - dioctyl ester of sodium sulfosuccinic acid | Aerosol™ OT Surface Active Agent | Cytec Industries, Process Chemicals, Unit of American Cyanamid, West Paterson, NJ |
| 3,23,40 | Alkylbenzene sulfonates and sulfates | anionic - sodium branched alkyl (C₁₂) benzene sulfonate | Polystep™ A-16 | Stepan Company, Nonhfield, IL |
| 4, 21 | Alkylbenzene sulfonates and sulfates | anionic - sodium dodecyl benzene sulfonate | Rhodocal™ DS-10 | Rhone-Poulenc, Surfactant & Specialty Division, Cranberry, NJ |
| 5 | Ethoxylated and polyethoxylated alkyl or aralkyl alcohol carboxylates | anionic - alkyl (C₁₂-C₁₅) (ethoxylated) carboxylate | Sandopan LS-24 Carboxylated Surfactant | Sandoz Chemicals Corp., Charlotte, NC |
| 6, 15 | N-acyltaurine derivatives | anionic - alkyl (C₁₈) sulfoamide sodium N-methyl-N-oleyl taurate | Igepon™ T-77 | GAF, New York, NY |
| 7 | Amophoteric alkyl carboxylates | amphoteric - cocamidopropyl betaine | Tego™ Betaine F-50 | Goldschmidt Chemical Corp., Hopewell, VA |
| 8,22, 53-60, Comp. Z-AC | Perfluoro-aliphatic anionic surfactants | anionic - ammonium perfluoroalkyl sulfonate | Fluorad™ FC-120 Fluoro-chemical Surfactant | 3M Company, Specialty Chemical Products Division, Maplewood, MN |
| 9, 11, 26, 33 | Perfluoro-aliphatic anionic surfactants | anionic - C₁₀F₂₁SO₃Li | | Made as described in U.S. Pat. No. 2,732,398 (Brice et al.) Example 2 |
| 10, 25 | Perfluoro-aliphatic anionic surfactants | anionic - C₈F₁₇SO₃Li | | Made as described in U.S. Pat. No. 2,732,398 (Brice et al.) Example 2 |
| 12, 31 | Polyethoxylated derivatives of straight or branched chain aliphatic sulfates | anionic -polyethoxylated alkyl (C₁₂) ether sulfate, ammonium salt | Steol CA-460 | Stepan Company, Northfield, IL |
| 13 | Perfluorinated radical substituted aliphatic anionic salts | anionic -fluorochemical surfactant | Zonyl™ FSE Fluoro-Surfactant | E. I. Du Pont de Nemours & Co., Chemicals Dept., Wilmington, DE |
| 14 | Isethionate derivatives | anionic - alkyl (C₁₃) sulfoester (SO₃⁻ Na⁺) | Igepon™ AC-78 | obtained from GAF, New York, NY |
| 16 | Ethoxylated and polyethoxylated alkyl or aralkyl alcohol carboxylates | anionic - polycthoxylated alkyl (C₁₀- C₁₆) carboxylates | Sandopan L8-HC Carboxylated Surfactant | Sandoz Chemicals Corp., Charlotte, NC |
| 17 | Straight or branched chain aliphatic sulfates and sulfonates | anionic - aliphatic sulfates | Hostastat HS-1 | Hoechst Celanese Corp., Colorants & Surfactants, Charlotte, NC |
| 18 | Glycinates | anionic sodium cocoyl sarcosinate (CO₂⁻ Na⁺) | Hampshire™ Hamposyl C-30 | Hampshire Chemical, Lexington, MA |
| 24 | Alkylbenzene sulfonates and sulfates | anionic - sodium linear alkyl (C₁₂) benzene sulfonate | Polystep™ A-15 | Stepan Company, Northfield, IL |
| 27 | Glycinates | amphoteric - dihydroxyethyl tallow glycinate | Mackam™ TM Amphoteric | The McIntyre Group Ltd., University Park, IL |
| 28 | Sulfosuccinates | anionic - alkyl (C₁₃) sulfosuccinates | Aerosol™TR Surface Active Agent | Cytec Industries, Process Chemicals, Unit of American Cyanamid, West Paterson, NJ |
| 29 | Amphoteric alkyl carboxylates | amphoteric - oleyl betaine | Mackam™ OB-30 Amphoteric | The McIntyre Group Ltd., University Park, IL |
| 30 | Amphoteric alkyl carboxylates | amphoteric -dicarboxylic coconut imidazoline derivative, sodium salt | Miranol™ 2CIB | Rhone-Poulenc, Surfactant & Specialty Division, Cranberry, NJ |
| 32 | Alkyl phosphate mono- or di-esters | alkyl (C₁₈) phosphate ester (PO₄⁻Na₂⁺) | Sipostat 0018 | Specialty Industrial Products Inc., Spartanburg, SC |
| 34, 50, 51, 52, Comp. U-Y | Perfluoro-aliphatic anionic surfactants | anionic - potassium perfluoroalkyl sulfonate | Fluorad™ FC-95 Fluoro-chemical Surfactant | 3M Company, Specialty Chemical Products Division, Maplewood, MN |
| 35 | Straight or branched chain aliphatic sulfates and sulfonates | amphoteric -cocamidopropyl hydroxysultaine | Mackam™ CBS-50 Amphoteric | The McIntyre Group Ltd., University Park, IL |
| 36 | Amphoteric alkyl carboxylates | amphoteric-N-coco-b-aminopropi onic acid | Mirataine™ AP-C | Rhone-Poulenc, Surfactant & Specialty Division, Cranberry, NJ |
| 37 | Alkylbenzene sulfonates and sulfates | anionic - sodium salt of dodecyl diphenyl ether disulfonate derived from propylene tetramer | Poly-Tergent™ 2EP | Olin Corp., Stamford, CT |
| 38 | Amphoteric alkyl carboxylates | amphoteric -oleamido betaine | Mackam™ HV Amphoteric | The McIntyre Group Ltd., University Park, IL |
| 39 | Alkyl phosphate mono- or di-esters | ethoxylated docecyl alcohol phosphate ester, sodium salt (PO₄⁻Na⁺) | Rhodafac™ MC-470 | Rhone-Poulenc, Surfactant & Specialty Division, Cranberry, NJ |
| 43,45 | Perfluoro-aliphatic anionic surfactants | anionic - potassium salt of a perfluoro-aliphatic sulfonamido carboxylate | Fluorad™ FC-129 Fluoro-chemical Surfactant | 3M Company, Specialty Chemical Products Division, Maplewood, MN |
| 44,46 | Perfluoro-aliphatic anionic surfactants | anionic- potassium salt of a perfluoro-aliphatic sulfonamido carboxylate | Fluorad™ FC-127 Fluoro-chemical Surfactant | 3M Company, Specialty Chemical Products Division, Maplewood, MN |
| 47, 48 | Perfluoro-aliphatic anionic surfactants | anionic - C₄F₉PO₃-(Li)₂ | | U.S. Pat. No. 2,732,398 (Brice et al.) Example 2 and U.S. Pat. No. 2,809,990 (Brown) |
| 49 | Perfluoro-aliphatic anionic surfactants | anionic - C₃F₇CO₂Li | | U.S. Pat. No. 2,732,398 (Brice et al.) Example 2 and U.S. Pat. No. 2,809,990 (Brown) |
| 61, Comp. AD, AF, AH | Perfluoro-aliphatic anionic acid | anionic - C₄F₉SO₃H | | U.S. Pat. No. 2,732,398 (Brice et al.) Example 2 and U.S. Pat. No. 2,809,990 (Brown) |
| 62, 63, Comp. AE, AG | Perfluoro-aliphatic anionic surfactants | anionic - an ammonium salt of an anionic perfluoro-aliphatic radical containing sulfonate | Fluorad™ Fluoro-chemical Surfactant | 3M Company, Specialty Chemical Products Division, Maplewood, MN |

| Ex. No. | Surfactant Class | Type - chemical description | Trade Name | Source/Address |
|---|---|---|---|---|
| Comp. A | Polyethoxylated alkyl phenol | nonionic -polyethoxylated octyl phenols | Triton™ X-100 | Union Carbide Chemical & Plastics Co., Industrial Chemicals Division, Danbury, CT |
| Comp. B | Polyethoxylated alkyl alcohol | nonionic-polyoxyethylene (23)lauryl ether | Brij™ 35 | ICI Americas Inc., Wilmington, DE |
| Comp. C | Block copolymers of polyethylene oxide and polypropylene oxide | nonionic - block copolymer of ethylene oxide and propylene oxide, ethylene diamine started (PEG/PPG/PEG block copolymer) | Tetronic™ 1502 Block Copolymer Surfactant | BASF Corp., Performance Chemicals, Parsippany, NJ |
| Comp. D | Polyethoxylated alkyl alcohol | nonionic -polyoxyethylene (4) lauryl ether | Brij™ 30 | ICI Americas Inc., Wilmington, DE |
| Comp. E | Block copolymers of polyethylene oxide and polypropylene oxide | nonionic -PEG/PPG/PEG block copolymer | Pluronic™ F38 Block Copolymer Surfactant | BASF Corp. Performance Chemicals, Parsippany, NJ |
| Comp. F | Polyethoxylated alkyl phenol | nonionic -polyethoxylated nonyl phenol | Iconol NP-70 | BASF Corp. Performance Chemicals, Parsippany, NJ |
| Comp. G | Block copolymers of polyethylene oxide and polypropylene oxide | nonionic -PEG/PPG/PEG block copolymer | Tetronic™ 908 Block Copolymer Surfactant | BASF Corp., Performance Chemicals, Parsippany, NJ |
| Comp. H | Perfluorinated radical containing polyethoxylated alcohol | nonionic -fluorochemical surfactant | Zonyl™ FSN Fluoro-Surfactant | E. I. Du Pont de Nemours & Co., Chemicals Dept., Wilmington, DE |
| Comp. I | Polyethoxylated alkyl alcohol | nonionic - C₁₁-C₁₅ secondary alcohol ethoxylate | Tergitol™ 15-S-40 Specialty Surfactant | Union Carbide Chemical & Plastics Co., Danbury, CT |
| Comp. J | Amine oxide | nonionic - lauryl dimethylamine oxide | Rhodamox™ LO | Rhone-Poulenc, Surfactant & Specialty Division, Cranberry, NJ |
| Comp.K | Polyethoxylated alkyl alcohol | nonionic -ethoxylated trimethylnonanol | Tergitol™ TMN-6 Specialty Surfactant | Union Carbide Chemical & Plastics Co., Danbury, CT |
| Comp. L | | ammonium xylene sulfonate | | Reutgers-Neese Chemical Co., State College, PA |
| Comp. M | Alkyl carboxylate | lithium stearate | | See Preparation in Comparative Examples M-N |
| Comp. N | Alkyl carboxylate | sodium stearate NF | | Witco, Organics Division, New York, NY |
| Comp. O, R | Perfluoro-aliphatic nonionics | nonionic - perfluoro-aliphatic radical containing sulfonamide | Fluorad™ FC-170 Fluoro-chemic al Surfactant | 3M Company, Specialty Chemical Products Division, Maplewood, MN |
| Comp. P, S | Perfluoro-aliphatic nonionics | nonionic - perfluoro-aliphatic radical containing sulfonamide | Fluorad™ FC-431 Fluoro-chemic al Surfactant | 3M Company, Specialty Chemical Products Division, Maplewood, MN |
| Comp. Q, T | Perfluoro-aliphatic nonionics | nonionic - perfluoro-aliphatic radical containing sulfonamide | Fluorad™ FC-171 Fluoro-chemical Surfactant | 3M Company, Specialty Chemical Products Division, Maplewood, MN |

### Examples 1-11 and Comparative Examples A-C

The anti-fog/anti-reflective compositions of Examples 1 through 11 and the comparative anti-reflective compositions of Comparative Examples A through C were prepared by adding the surfactants described in Table 1 in the amounts given in Table 2 to a dispersion containing 1.75 weight percent silica prepared by dilution of "Remasol SP-30 Sodium Stabilized Silica Sol" (supplied as a 30 percent solution, particle size 70 Å, pH 10) commercially available from Remet Corp., Chadwicks, NY, in deionized water. A glycidoxypropyltrimethoxysilane (GPS) coupling agent, commercially available as "A-187" from Union Carbide Chemical & Plastics Company, Danbury, CT, was added to the dispersion of Example 11 at a concentration of 0.17 percent by weight. The compositions were coated on both sides of a 0.18 mm (7 mil) thick flame treated polyethylene terephthalate (PET) film using a roll coater with an air knife to adjust thickness of the dried coating to a violet to slightly blue hue when viewed by reflected light (approximately 1,000 to 1,200 Å). The coated film was immediately passed into a forced air dryer at a temperature of 77°C. The dwell time in the oven was less than 2 minutes. The samples were evaluated for fog resistance the day the coated film samples were made ("initial fog") and again after aging 12 days in storage but with the surfaces exposed to ambient conditions of approximately 23°C and 50 percent relative humidity. Fogging was evaluated by breathing directly on the film with the film held approximately 2.5 cm from the mouth. Initial fog was determined subjectively as "excellent," "good," "okay," and "poor" depending on the relative ability to see through the film after breathing upon it. The following numerical ratings were used for the 12-day evaluation: "1" means the coated film fogs like the uncoated film; "2" means the coated film fogs after 1-2 consecutive breaths; "3" means the coated film had a light fog after 3 consecutive breaths; "4" means the coated film had a very light fog after 4 consecutive direct breaths; and "5" means the coated film resisted fogging after 5 consecutive direct breaths. The results are reported in Table 2. All of the coated films, when visually compared to uncoated film and held up to a textured beige surface, were significantly more transparent and anti-reflective.

**TABLE 2**

| **Example Number** | **Surfactant Concentration (weight %)** | **Initial Fog** | **Fog After 12 days Aging** |
|---|---|---|---|
| Comp. A | 0.150 | good | 2 |
| Comp. B | 0.014 | good | 2 |
| Comp. C | 0.015 | poor | 2 |
| 1 | 0.015 | excellent | 5 |
| 2 | 0.010 | excellent | 4 |
| 3 | 0.015 | good | 4 |
| 4 | 0.015 | good | 5 |
| 5 | 0.018 | good | 5 |
| 6 | 0.015 | good | 4 |
| 7 | 0.014 | good | 4 |
| 8 | 0.015 | okay | 5 |
| 9 | 0.015 | good | 5 |
| 10 | 0.035 | good | 5 |
| 11 | 0.040 | good | 5 |

Discussion of Results: The films which were coated with compositions containing the surfactants in Comparative Example A and B were resistant to fogging initially but were easily fogged after aging for only 12 days. The film which was coated with a composition containing the surfactant used in Comparative Example C was easily fogged initially and after aging. The eleven films which were coated with compositions containing the variety of surfactants in Examples 1-11 had good to excellent resistance to fogging initially and after aging for 12 days.

### Examples 12-13 and Comparative Examples D-I

The anti-fog/anti-reflective compositions of Examples 12 and 13 and the comparative anti-reflective compositions of Comparative Examples D through I were prepared by adding the types of surfactants described in Table 1 in the amounts given in Table 3 to a dispersion containing 1.25 weight percent silica prepared by dilution of "Nalco 2326 Colloidal Silica as SiO₂" (supplied at 15 weight percent, particle size 50 angstroms), commercially available from Nalco Chemical Company, Naperville, IL, in deionized water. A silane coupling agent, glycidoxypropyltrimethoxysilane (GPS), commercially available as "A-187" from Union Carbide Chemical & Plastics Company, was also added to the composition of Comparative Examples D-I and Example 12. Another silane coupling agent commercially available as "A1230," a proprietary nonionic silane dispersing agent from Union Carbide Chemical & Plastics Company, was added to the composition of Example 13. The amounts of silane coupling agents are described in Table 3. The compositions were allowed to sit for approximately 1 hour prior to coating. The compositions were coated by hand on one side only of a 0.18 mm (7 mil) thick PET film using a number 8 Meyer bar. The coated films were dried in an oven at 85°C for approximately 5 minutes. The coated side of the film was evaluated for fogging approximately 24 hours after coating. Fogging was determined using the fogging test described for 12 day fog in Examples 1-11. The results are reported in Table 3.

**TABLE 3**

| Example Number | Silane Concentration (Weight %) | Surfactant Concentration (Weight %) | Initial Fog |
|---|---|---|---|
| Comp. D | 0.15 | 0.150 | 1 |
| - Comp. E | 0.12 | 0.120 | 1 |
| Comp. F | 0.12 | 0.120 | 2 |
| Comp. G | 0.10 | 0.120 | 1 |
| Comp. H | 0.12 | 0.096 | 1 |
| Comp. I | 0.12 | 0.120 | 1 |
| 12 | 0.10 | 0.020 | 5 |
| 13 | 0.12 | 0.017 | 4 |

Discussion of results: Even at the relatively high levels used the surfactants of Comparative Examples D-I were not able to produce anti-fog coatings.

### Examples 14-18

The anti-fog/anti-reflective compositions of Examples 14a through 14c, 15a through 15c, 16a through 16c, 17a through 17c, and 18a through 18c were made by adding the surfactants given in Table 1 at the concentration indicated in Table 4 to 50 g of a dispersion containing 1.5 weight percent silica prepared by dilution of "Remasol SP-30 Sodium Stabilized Silica Sol" in deionized water. The compositions were coated on both sides of a flame treated 20 x 30 cm x 0.18 mm (7 mil) thick PET film using a number 6 Meyer bar as follows. The first side was coated and immediately dried at 100°C for 1-2 minutes. Next the second side was coated and dried at 100°C for 1 to 2 minutes. The coated films were evaluated for fog approximately 2 days after coating using the test described for 12 day fog in Examples 1-11. The results are reported in Table 4.

**TABLE 4**

| Example Number | Surfactant Concentration (Weight %) | Fog |
|---|---|---|
| 14 a | 0.0080 | 4 |
| 14 b | 0.0120 | 4 |
| 14 c | 0.0200 | 5 |
| 15 a | 0.0080 | 5 |
| 15 b | 0.0120 | 5 |
| 15 c | 0.0200 | 5 |
| 16 a | 0.0080 | 4 |
| 16 b | 0.0140 | 4 |
| 16 c | 0.0200 | 4 |
| 17 a | 0.0500 | 4 |
| 17 b | 0.0130 | 4 |
| 17 c | 0.0063 | 4 |
| 18 a | 0.0080 | 5 |
| 18 b | 0.0040 | 5 |
| 18 c | 0.0020 | 4 |

Discussion of results: The coatings of Examples 14 (a-c) and 18 (a-c) were spotty. The coated films of Examples 15 and 16 had anti-fog properties and were anti-reflective. The coating compositions of Examples 14a-c, 15a-c, 16a-c and 18a-c provided an anti-reflective surface, as determined using the visual comparison for anti-reflection described in Examples 1-11. The results of visual inspection of the anti-reflective property of the film of Example 17a were poor, but those of 17b and 17c were good, presumably due to the lower level of surfactant.

### Examples 19-25

The anti-fog/anti-reflective compositions of Examples 19 through 25 were made by adding the surfactants given in Table 1 at a concentration of 0.015 weight percent to a dispersion containing 2.5 weight percent silica prepared by dilution of "Remasol SP-30 Sodium Stabilized Silica Sol" and 0.25 weight percent of the coupling agent, GPS, commercially available as "A-187" from Union Carbide Chemical & Plastics Company. The compositions were coated on both sides of a flame treated 20 x 30 cm x 0.18 mm (7 mil) thick PET film as described for Examples 14-18. An initial determination of the tendency of the film to fog was made as described below. The remaining samples were hung in a recirculated oven held at 49°C. Film samples were removed at 6, 10, 16, 20 and 26 day intervals and evaluated for the tendency to fog using the following fog test. A new sample was tested at each time period.

**Fog Test**: Individual film samples were held over a steam source for approximately 5 seconds. The "steam" (i.e., saturated water vapor) source was a container of boiling deionized water which was equipped with an inverted funnel that allowed the steam to exit approximately 10-13 cm above the liquid level through an opening which is approximately 1.3 cm in diameter. The "steam" temperature was approximately 55°C. The film sample was held approximately 5-8 cm above the steam exit. The results were determined subjectively as "good" (the film did not fog), "trace" (a slight amount of fog was detected), and "poor" (the film fogged) on the relative ability to see through the film during the test. The results are reported in Table 5.

**TABLE 5**

| **Ex. No.** | **Initial** | **Day 6** | **Day 10** | **Day 16** | **Day 20** | **Day 26** |
|---|---|---|---|---|---|---|
| 19 | good | good | -¹ | good | -¹ | good |
| 20 | good | good | -¹ | good | -¹ | good |
| 21 | good | good | -¹ | good | -¹ | good |
| 22 | good | good | -¹ | trace | -¹ | -¹ |
| 23 | good | -¹ | good | -¹ | good | -¹ |
| 24 | good | -¹ | good | -¹ | good | -¹ |
| 25 | good | -¹ | good | -¹ | good | -¹ |
| "-¹" indicates sample was not tested at this time interval. | | | | | | |

Discussion of results: The coated films had good anti-fog properties initially and after at least 20 days of aging, except for the coated film of Example 22 which had a trace of fog after 16 days of aging. All of the coating compositions provided an anti-reflective surface, as determined using the test for anti-reflection described in Examples 1-11.

### Example 26

The anti-fog/anti-reflective compositions of Examples 26a through 26d were prepared using the surfactant of Example 9 in a 1.25 weight percent dispersion of "Nalco 1042 Colloidal Silica as SiO₂" (14.5 weight percent, particle size 20 nanometers, pH of 2.8), commercially available from Nalco Chemical Company, Naperville, IL, and 0.125 weight percent of the silane coupling agent, GPS, commercially available as "A-187" from Union Carbide Chemical & Plastics Company. The surfactant was added to 50 g aliquots of the sol at the concentrations indicated in Table 6. The anti-fog/anti-reflective compositions were allowed to sit for 4 hours to allow the silane to hydrolyze. It was observed that this resting time resulted in improved coating quality. The compositions were coated on both sides of a 20 x 30 cm x 0.18 mm (7 mil) thick PET film as described for Examples 14-18. The coated films were dried in an oven at 100°C for approximately 1-2 minutes. The anti-fog property of the coated films was evaluated using the Fog Test described in Examples 19-25. The anti-reflective property of the film was determined by holding the sample up to the light and against a textured beige colored surface and evaluating its relative light transmission as "good," meaning a significant improvement over uncoated film; "fair," meaning a slight improvement over uncoated film; and "poor," meaning about the same as uncoated film. The results are reported in Table 6.

**TABLE 6**

| **Example Number** | **Surfactant Concentration (weight %)** | **Anti-fog** | **Anti-reflection** |
|---|---|---|---|
| 26 a | 0.03 | excellent | good |
| 26 b | 0.05 | excellent | good |
| 26 c | 0.07 | excellent | good |
| 26 d | 0.09 | excellent | good |

Discussion of results: The coated films had excellent anti-fog properties, but the higher the level of surfactant, the better the resistance to fogging. However as the concentration of the surfactant was increased, the anti-reflective property became poorer, indicating an optimal concentration of about 0.03 weight percent for this surfactant.

### Examples 27-32

The anti-fog/anti-reflective compositions of Examples 27a through 27c, 28a through 28c, 29a through 29c, 30a through 30c and 31a through 31c were prepared by adding the surfactants given in Table 1 in the amounts given in Table 7 to 50g of a 1.75 weight percent dispersion of "Remasol SP-30 Sodium Stabilized Silica Sol" in deionized water. The compositions were coated on both sides of a corona discharge treated 20 x 30 cm x 0.18 mm (7 mil) thick PET film as described for Examples 14-18. The initial fog was evaluated using the Fog Test described in Examples 19-25 except that the following rating scale was used: "0" means no fog, "1" means minimal slight haze, "2" means medium fog, and "3" means heavy fog or the same as an uncoated polyester film. The coating quality was determined visually and the following rating scale was used: "very good" means virtually no coating defects, a uniform finish; "good" means only minor coating inconsistencies; "okay" means some coating inconsistencies; "spots" means visible non-wet spots were observed (indicating a need to vary the surfactant concentration or add a wetting agent); and "non-wets" means many coating inconsistencies (indicating the need for a higher concentration of surfactant or the addition of a wetting agent to the coating composition). The results are reported in Table 7. Anti-reflection of the coated films was measured qualitatively by visual observation as described in Example 26. The results are also reported in Table 7.

**TABLE 7**

| **Ex. No.** | **Amount of 2% surfactant solution added (g)** | **Surfactant Concentration (weight %)** | **Coating Quality** | **Anti-reflection** | **Initial Fog** |
|---|---|---|---|---|---|
| 27a | 0.20 | 0.008 | okay | fair | 0 |
| 27b | 0.35 | 0.014 | spots | fair | 0 |
| 27c | 0.50 | 0.020 | spots | fair | 0 |
| 28a | 0.20 | 0.008 | non-wets | fair | 0 |
| 28b | 0.35 | 0.014 | non-wets | fair | 0 |
| 28c | 0.50 | 0.020 | good | good | 0 |
| 29a | 0.20 | 0.008 | spotty | fair | 0 |
| 29b | 0.35 | 0.014 | okay | good | 0 |
| 29c | 0.50 | 0.020 | good | good | 0 |
| 30a | 0.20 | 0.008 | good | good | 0 |
| 30b | 0.35 | 0.014 | good | good | 0 |
| 30c | 0.50 | 0.020 | good | good | 0 |
| 31a | 0.20 | 0.008 | good | good | 0 |
| 31b | 0.35 | 0.014 | good | good | 0 |
| 31c | 0.50 | 0.020 | very good | good | 0 |
| 32a | 0.20 | 0.008 | good | good | 0 |
| 32b | 0.35 | 0.014 | good | fair | 0 |
| 32c | 0.50 | 0.020 | good | fair | 0 |

### Discussion of Results:

The results indicate that within this set of surfactants better wetting is achieved at higher levels of surfactant. Example 27(a)-(c) showed an improvement and Examples 28(a)-(c), 29(a)-(c), 30(a)-(c), 31(a)-(c), and 32(a)-(c) showed a significant improvement in the anti-reflective property. All samples had good initial anti-fog properties.

### Examples 33-41 and Comparative Examples J-L

The anti-fog/anti-reflective compositions of Examples 33 through 42 and the anti-reflective compositions of Comparative Examples J through L were prepared as follows. A master batch of colloidal silica was made from "Remasol SP-30 Sodium Stabilized Silica Sol (30 percent solution)," GPS, commercially available as "G6720" from Huls, Piscataway, NJ, and deionized water in the following amounts:

| **Material** | **Amount (g)** |
|---|---|
| Deionized Water | 142,600 |
| Colloidal silica | 8,840 |
| GPS | 265 |

The materials were added in the order listed with a minimum of 5 minutes of mixing between additions. The mixture was allowed to stir overnight. The surfactant listed in Table 1 in the amount and concentration (in deionized water) described in Table 8 was added to 18,900 gram aliquots of the master batch solution. The compositions were coated onto a 30.5 cm wide, 0.18 cm (7 mil) thick corona discharge treated PET film using a roll coater and air knife as described in Examples 1-11. The air knife was operated at a pressure of approximately 5-13 cm of water column. The setting varied with each composition in order to attain the desired thickness of coating which after drying had a violet to slightly blue hue. The coated films were immediately passed into a forced air oven at a temperature of 77°C. The dwell time in the oven was less than 2 minutes. The second side was coated in a similar manner. All of the coating compositions provided an anti-reflective surface, as determined using the test for anti-reflection described in Example 26. The coating quality was evaluated visually and the following rating scale was used: "excellent" means virtually no coating defects, a uniform finish; "very good" and "good" indicate only minor coating inconsistencies; "okay" means some coating inconsistencies; and "poor" means many coating inconsistencies. The results are reported qualitatively in Table 8.

**TABLE 8**

| Ex. No. | Amount of surfactant solution added (g of percent by wt. solution) | Surfactant Concentration (weight %) | Coating Quality |
|---|---|---|---|
| 33 | 56.8 (10%) | 0.030 | excellent |
| 34 | 113.4 (5%) | 0.030 | excellent |
| 35 | 66.5 (4%) | 0.014 | very good |
| 36 | 94.6 (4%) | 0.020 | good |
| 37 | 71.0 (4%) | 0.015 | good |
| 38 | 94.6 (4%) | 0.020 | very good |
| 39 | 94.8 (4%) | 0.020 | okay |
| 40 | 71.0 (4%) | 0.015 | excellent |
| 41 | 142.0 (2%) | 0.015 | very good |
| Comp. J | 37.8 (4%) | 0.008 | good |
| Comp. K | 56.7 (4%) | 0.012 | good |
| Comp. L | 66.2 (4%) | 0.014 | good |

The coated films were aged using an Aging Test described below.

**Aging Test:** Multiple coated film samples were cut, 5 cm x 15 cm. Great care was taken to keep the coated film samples from becoming contaminated. Personnel wore cotton gloves and samples were not placed in packaging materials which could result in surface contamination. A magazine was made from polystyrene foam core/paper board, with razor slits approximately 1.3 cm deep and 1.3 cm apart cut into one edge of the board. The coated film samples were placed in the magazine so that adjacent samples were not touching and so that substantially the entire surface area of the sample was exposed to the environment. The sample loaded magazines were placed in an oven capable of totally recirculating and recycling air. The oven conditions were: 1) a recirculation rate of 1.67 volumes/minute, 2) a temperature of 30°C, and 3) a relative humidity of 60 percent. It was possible that in some samples an increase in fogging was a result of surface contamination due to the air quality inside the oven. Therefore, relative differences may be more important than the actual values. Film samples were removed at regular time intervals of 7, 14, 28, 56 and 84 days and evaluated by the Wetting Test described below. The "initial" sample was evaluated after standing at room temperature for 24 to 48 hours after coating.

**Wetting Test:** Each film sample was conditioned at 23°C and 50 percent relative humidity for a minimum of 8 hours before and during testing. Care was taken to ensure that the film samples were not contaminated and that exposure to the environment did not result in decreased wetting. The film samples were placed on a clean flat horizontal surface with the side to be tested up. A 3 microliter drop of deionized and distilled water containing 0.07% by weight "Wool Fast Brilliant Red R.L. Dye," commercially available from Pylam, Garden City, NY, from an accurate syringe was gently placed on the surface by holding the syringe vertically and just touching the drop to the surface so that the drop did not fall and impact the surface. The drop was allowed to spread to its maximum extent and completely dry. The diameter of the drop was determined by placing the film over a paper with premeasured circles of varying diameters. The average drop diameter was recorded. The dye did not interact with the surfactant system being tested, as verified by comparing the results with results without the dye. The results of the Wetting Test are reported in Table 9.

**TABLE 9**

| | **Wetting Value after Aging** | | | | | |
|---|---|---|---|---|---|---|
| **Ex. No.** | **Initial (mm)** | **Day 7 (mm)** | **Day 14 (mm)** | **Day 28 (mm)** | **Day 56 (mm)** | **Day 84 (mm)** |
| 33 | 9.0 | -¹ | 8.1 | -¹ | 4.3 | 3.9 |
| 34 | 8.6 | 9.1 | 6.6 | 5.0 | 4.6 | 3.8 |
| 35 | 8.8 | 8.5 | 5.5 | 4.4 | 4.0 | 3.9 |
| 36 | 8.0 | 7.5 | 6.7 | 4.7 | 4.3 | 4.0 |
| 37 | 8.3 | 8.5 | 6.0 | 6.1 | 4.2 | - |
| 38 | 8.6 | 7.4 | 5.4 | 4.9 | 4.0 | 3.8 |
| 39 | 9.2 | 7.0 | 5.8 | 4.4 | 4.4 | 3.8 |
| 40 | 8.7 | 7.0 | 5.8 | 4.4 | 4.4 | 3.8 |
| 41 | 7.9 | 6.8 | 5.4 | 4.6 | 4.0 | 3.8 |
| Comp. J | 8.7 | 4.3 | 3.9 | 3.8 | 3.8 | 3.6 |
| Comp. K | 8.6 | 5.6 | 4.4 | 4.0 | 3.8 | 3.6 |
| Comp. L | 8.9 | 5.1 | 4.6 | 3.9 | 3.9 | 3.6 |
| "-¹" means sample was not tested at that time interval. | | | | | | |

Discussion of results: Uncoated PET film had a wetting value of 2.75 mm for comparison. Actual breathing tests such as those described in Examples 1-11 indicated that once the wetting values fall below about 4.1 mm the fogging was unacceptable for use in a surgical mask application. The surfactants of the present invention produced coated films with acceptable wetting values (indicating a resistance to fogging) beyond day 28 and several beyond day 56 in this accelerated aging test. The coated films of Comparative Examples K-L lost their anti-fog properties between day 14 and day 28 and the coated film of Comparative Example J by day 14.

### Aging in Sealed Environments

In order to better understand the aging properties of the various surfactants and whether environmental contamination of the films decreases their anti-fog property, coated films from Examples 33-35, 38, 40-41, and Comparative Example J were conditioned in a 25°C/50 % relative humidity environment overnight (approximately 12 hours) and were placed in two sets of sealed jars. Separate jars were used for coated films containing different surfactants. Jars in Set 1 (Dry) contained only the coated films, while jars in Set 2 (Wet) contained the coated films and a small vial of deionized water sufficient to ensure it did not completely evaporate. The water in the vial did not contact the films directly, but served as a source of high humidity. The jars were placed in a 40°C oven and films were periodically withdrawn and tested for Wetting as described above. The results are reported in Table 10 for dry aging and Table 11 for wet aging. The coated films containing the surfactants of the present invention performed extremely well as durable anti-fogging agents when maintained in a sealed chamber. The fogging characteristics did not significantly decrease even after 56 days. The wetting of Comparative Example J fell rapidly even in a sealed container. While not being bound to any theory, the results indicated that loss of anti-fog properties may be due to environmental surface contamination of the coated films. Loss of anti-fog properties in the comparative example appeared to be due to other causes since the anti-fog property decreased very rapidly even in a sealed container.

**TABLE 10**

| | **Set 1 "Dry" Wetting Value after Aging** | | | | | |
|---|---|---|---|---|---|---|
| **Ex. No.** | **Initial (mm)** | **Day 7 (mm)** | **Day 14 (mm)** | **Day 28 (mm)** | **Day 56 (nm)** | **Day 84 (mm)** |
| 33 | 8.7 | 8.2 | 7.4 | 9.0 | 9.6 | 8.7 |
| 34 | 8.0 | 7.2 | 7.4 | 8.9 | 8.2 | 9.3 |
| 35 | 8.8 | 7.6 | 7.2 | 9.1 | 9.5 | 7.8 |
| 38 | 8.6 | 6.3 | 6.9 | 8.1 | 8.9 | 8.5 |
| 40 | 8.7 | 7.4 | 7.7 | 8.6 | 9.1 | 8.2 |
| 41 | 7.9 | 6.6 | 6.3 | 8.9 | 8.8 | 8.2 |
| Comp. J | 8.7 | 4.7 | 4.5 | 4.8 | 3.8 | 4.0 |

**TABLE 11**

| | **Set 2 "Wet" Wetting Value after Aging** | | | | |
|---|---|---|---|---|---|
| **Example Number** | **Initial (mm)** | **Day 7 (mm)** | **Day 14 (mm)** | **Day 28 (mm)** | **Day 56 (mm)** |
| 33 | 8.7 | 8.1 | 8.3 | 8.5 | 7.5 |
| 34 | 8.0 | 8.2 | 7.6 | 8.0 | 7.8 |
| 35 | 8.8 | 7.2 | 7.4 | 8.4 | 6.9 |
| 38 | 8.6 | 7.1 | 6.6 | 6.7 | 6.7 |
| 40 | 8.7 | 8.4 | 8.2 | 7.7 | 7.8 |
| 41 | 7.9 | 7.6 | 7.3 | 8.1 | 7.3 |
| Comp. J | 8.7 | 4.4 | 4.1 | 3.9 | 4.1 |

### Comparative Examples M-N

### Preparation of Surfactant

Lithium stearate was prepared by: a) dissolving 2.2 grams of lithium hydroxide monohydrate commercially available from Fisher Scientific, Pittsburgh, PA, in approximately 60 mL of water and heating to about 80°C until the lithium hydroxide was completely dissolved; b) heating 142 grams of stearic acid commercially available from Fisher Scientific, to about 80°C to melt the stearic acid and then adding about 10 mL of isopropyl alcohol commercially available from Fisher Scientific, while stirring; and c) adding part a) to part b) while still hot, stirring to form a loose dispersion, continuing to stir for 10-15 minutes while allowing the dispersion to cool. The resulting mass was washed twice by adding about 20 ml of warm water and filtering through a paper filter and repeating the sequence. The washed residue was pressed in the filter to squeeze out the excess wash water and was air-dried at about 45°C until it reached a constant weight. A loose fine powder was obtained.

### Preparation of Anti-reflective Coated Films

A 2 weight percent solution of surfactant was prepared by dissolving 1 gram of surfactant in 49 grams of deionized water. A master batch of silica sol with a silica solids concentration of 1.75 weight percent was prepared by adding 46.67 grams of "Remasol SP-30 Sodium Stabilized Silica Sol" (30 percent solution) to 753.3 grams deionized water. The anti-reflective composition of Comparative Example M was prepared by mixing the surfactant concentrate into 50 grams of the dilute silica dispersion. The anti-reflective composition of Comparative Example N was prepared similarly, except that the surfactant described in Table 1 was used in place of lithium stearate in the amount and concentration given in Table 12. The compositions were coated as described in Examples 14-18. The coated films were aged in an oven as described in Examples 33-42 and evaluated using the Wetting Test also described in Examples 33-42. Three coated films from Example 34 were reevaluated for comparison. The results are reported in Table 13.

**TABLE 12**

| Example Number | Amount of 2% surfactant solution added (g) | Surfactant Concentration (weight %) |
|---|---|---|
| Comp. M | 0.35 | 0.014 |
| Comp. N | 0.50 | 0.020 |

**TABLE 13**

| **Ex. No.** | **Wetting Value after Aging** | | | | |
|---|---|---|---|---|---|
| | **Initial (mm)** | **Day 7 (mm)** | **Day 14 (mm)** | **Day 28 (mm)** | **Day 35 (mm)** |
| Comp. M | 8.64 | 3.84 | 3.66 | 3.29 | 3.29 |
| Comp. N | 8.46 | 4.01 | 3.56 | 3.29 | 3.29 |
| 34 | 8.55 | - | 6.37 | - | 4.20 |
| "-" indicates sample was not evaluated at this time interval. | | | | | |

Discussion of results: The results clearly illustrated that the stearate salt surfactants did not provide a durable anti-fog coating. These results were in contrast to the carboxylate of Example 5 and illustrated the importance of including an additional polar substituent when the surfactant is a carboxylate.

### Example 42

An anti-fog/anti-reflective coating composition was prepared containing:

| **Component** | **Amount in Weight %** |
|---|---|
| Deionized water | 97.98 |
| "Remasol SP-30 Sodium Stabilized Silica Sol (30 percent solution)" from Remet Corporation | 1.83 (as silica) |
| GPS commercially available as "A-187" from Union Carbide Chemical & Plastics Company | 0.18 |
| "Fluorad™ FC-95 Fluorochemical Surfactant" from 3M Company | 0.015 |

The composition was stirred overnight and coated onto a flame treated 0.1 mm polyethylene terephthalate (PET) film according to the method described in Examples 1-11. The coating quality was evaluated as described in Examples 33-41 and found to be "excellent." The coated film was also evaluated for anti-fogging using the Wetting Test described in Examples 33-41. Both sides of the coated film had a 7.2 mm average drop diameter when evaluated at the "initial" time interval. The coated film and an uncoated PET film were evaluated for anti-reflective properties using a "Perkin-Elmer Spectrophotometer Model 552A" commercially available from Coleman Instrument Division, Oakbrook, IL. The percent transmission of the coated and uncoated film are reported in Table 14.

**TABLE 14**

| Percent Transmission | | | | | |
|---|---|---|---|---|---|
| | **Wave Length of Incident Light** | | | | |
| **Example Number** | **500 nm (%)** | **550 nm (%)** | **600 nm (%)** | **650 nm (%)** | **700 nm (%)** |
| 42 | 95.8 | 97.0 | 97.6 | 98.2 | 98.0 |
| Uncoated film | 84.0 | 86.0 | 86.0 | 86.0 | 86.0 |

The results indicated that the coated film of Example 42 had both exceptional anti-fog properties, as shown by the large average drop diameter, and anti-reflective properties, as shown by the 11-12.2 percent increase in light transmission over the uncoated film.

### Examples 43 to 49 and Comparative Examples O to T

The anti-fog/anti-reflective coating compositions of Examples 43-49 and the anti-reflective coating compositions of Comparative Examples O-T were prepared using a stock solution comprising 3,437 grams (g) of deionized water, 2.0 g of concentrated ammonium hydroxide, 326 g of "Nalco 2326 Colloidal Silica" (Nalco Chemical Co.), and 4.24 g of 3-aminopropyltriethoxysilane (APS) coupling agent. 0.1 g amounts of the various surfactants described in Table 1 were added to 200 g portions of the stock solution to prepare each coating composition. To the coating compositions of Examples 45, 46, and 48, and Comparative Examples R, S and T, 0.6 g of a 10% aqueous solution of "Triton X-100," a wetting agent commercially available from Union Carbide Chemical & Plastics Co., was also added as indicated in Table 15, hereinbelow.

Each coating composition was applied to both sides of a polyvinylidene chloride primed 0.1 mm thick polyethylene terephthalate film using a number 6 wire-wound rod (Meyer bar) to provide a coating about 1,000 Å thick on each side. The coated films were dried at 100°C for about 1 minute on each side and allowed to cool to room temperature. Each coated film was then evaluated for its anti-fogging properties using the following test procedure: A test apparatus comprising a laboratory stirring hot plate with a variable temperature control, a 1,000 milliliter (ml) glass beaker containing 500 ml of water, a 10 cm (4 inch) diameter polypropylene funnel placed in an inverted position on the flared rim of the beaker, and a 10 cm (4 inch) polypropylene tube secured to the distal end of the funnel discharge spout was constructed. The water was heated to boiling with stirring thereby generating a "steam" jet from the tube secured to the funnel. The "steam" temperature approximately 7.6 cm (3 inches) above the distal end of the tube was about 55°C. Coated films measuring 25.4 cm x 30.5 cm (10 in. x 12 in.) were passed through the "steam" jet approximately 7.6 cm (3 inch) above the tube outlet with a total exposure time of about 1 second. The coated substrates were then observed to determine whether a fog of condensed water vapor droplets had formed, significantly reducing the transparency of the substrate such that it could not be readily seen through. The results are reported in Table 15 along with the time required for samples that were not completely fogged to dry. The "transmittance" (i.e., the percentage point increase in the transmission of the coated substrate as compared to an uncoated substrate) was measured using ASTM Test Method D 1003-61, Procedure A (as reapproved in 1988). The results are also reported in Table 15.

**TABLE 15**

| **Example Number** | **Wetting Agent** | **Fog Formation** | **Drying Time (Seconds)** | **Transmittance (Percentage Point Increase)** |
|---|---|---|---|---|
| 43 | No | No | 14 | NM |
| 44 | No | No | 30 | NM |
| 45 | Yes | No | 24 | 6.0 |
| 46 | Yes | No | 20 | 3.9 |
| 47 | No | Partial | 14 | 7.1 |
| 48 | Yes | No | 8 | NM |
| 49 | No | No | 11 | NM |
| Comp. O | No | Yes | NT | NM |
| Comp. P | No | Yes | NT | NM |
| Comp. Q | No | Yes | NT | NM |
| Comp. R | Yes | Yes | NT | NM |
| Comp. S | Yes | Yes | NT | NM |
| Comp. T | Yes | Yes | NT | NM |
| NT=Not tested because substrate completely fogged. | | | | |
| NM=Not measured. | | | | |

The data in Table 15 illustrates the benefit of a coating composition that comprises an inorganic metal oxide (silica) and a surfactant of the present invention (i.e., either a potassium salt of a perfluoroaliphatic sulfonamido carboxylate compound or a lithium salt of an anionic perfluoroaliphatic radical-containing compound) in providing anti-reflection and anti-fogging properties to a film coated therewith. Coating compositions utilizing the nonionic fluorochemical compounds of Comparative Examples O, P, Q, R, S and T did not exhibit anti-fogging properties.

### Examples 50-52

### Comparative Examples U-Y

The anti-fog/anti-reflective coating compositions for use in Examples 50-52 and the anti-reflective coating compositions for use in Comparative Examples U-Y were prepared as described in Examples 43-49. The surfactant used in each composition was "Fluorad™ FC-95 Fluorochemical Surfactant" from 3M Company. Use of the wetting agent, "Triton X-100", is indicated in Table 16. Each composition was applied to both a polyvinylidene chloride (PVDC) primed 0.1 mm thick polyethylene terephthalate (PET) film (Examples 50 and 52 and Comparative Examples U and W) and an unprimed 0.1 mm thick PET film (Example 51 and Comparative Examples V, X and Y) using the method described for Examples 43-49 and using either a number 6 (Comparative Examples U, V, W and X) or a number 7 (Examples 50-52 and Comparative Example Y) wire wound rod (Meyer bar), as indicated in Table 16. The coated films were dried and the fogging evaluated as described in Examples 43-49. The results are reported in Table 16.

**TABLE 16**

| **Example Number** | **Primed Film** | **Wetting Agent** | **Meyer Bar Number** | **Fog** |
|---|---|---|---|---|
| Comp. U | yes | no | 6 | yes |
| 50 | yes | no | 7 | no |
| Comp. V | no | no | 6 | yes |
| 51 | no | no | 7 | no |
| Comp. W | yes | yes | 6 | yes |
| 52 | yes | yes | 7 | no |
| Comp. X | no | yes | 6 | yes |
| Comp. Y | no | yes | 7 | yes |

Comparative Examples U-X illustrate that films coated with a number 6 Meyer bar fogged. The coatings reproduced in Comparative Examples U-X appeared thin since the coatings had a gold hue. Therefore, in Examples 50-52 a number 7 Meyer bar was used to give a thicker coating. The coated films made using the number 7 Meyer bar were good anti-fog films. The results in Table 16 show that on either PVDC primed PET or unprimed PET as the coating thickness became thinner the film exhibited fogging. The reason for this was not clear. In addition, the presence of the wetting agent seemed to cause the fogging of the coated films to increase as can be seen by comparing the fogging results of Comparative Example Y to Example 51.

Comparative Examples U and V and examples 50 and 51 were replicated (again using a number 6 Meyer bar) except the "Fluorad™ FC-95 Fluorochemical Surfactant" concentrations were adjusted from 0.05 weight percent to 0.04 weight percent and 0.02 weight percent. The fogging results were exactly the same as the results shown in Table 16 for Comparative Examples U and V and Examples 50 and 51 demonstrating that decreasing the amount of surfactant did not improve fogging.

### Examples 53-60

### Comparative Examples Z-AC

Coating compositions were prepared as described for Examples 43-49. In one batch the silane coupling agent, aminopropyltriethoxysilane (APS), was omitted (Examples 53-56). In another batch the silane coupling agent, APS, was replaced with glycidoxypropyltrimethoxysilane (GPS), commercially available as "A-187" from Union Carbide Chemical & Plastics Company (Examples 57-60). The surfactant used in all compositions was "Fluorad™ FC-120 Fluorochemical Surfactant" from 3M Company. Batches of the above-described compositions were made with and without the wetting agent, "Triton™ X-100" from Union Carbide Chemical & Plastics Co. Each coating composition was applied to both sides of an unprimed 0.1 mm thick PET film using either a number 6 (Examples 53, 55, 57 and 59, and Comparative Examples Z and AB) or a number 7 wire wound rod (Meyer bar) (Examples 54, 56, 58 and 6, and Comparative Examples AA and AC). The coatings were dried and the fogging evaluated as described for Examples 43-49. The results of the fog test are given in Table 17.

**TABLE 17**

| **Example Number** | **Silane Coupling Agent Type** | **Wetting Agent** | **Meyer Bar Number** | **Fog** |
|---|---|---|---|---|
| Comp. Z | APS | no | 6 | yes |
| Comp. AA | APS | no | 7 | yes |
| Comp. AB | APS | yes | 6 | yes |
| Comp. AC | APS | yes | 7 | yes |
| 53 | none | no | 6 | no |
| 54 | none | no | 7 | no |
| 55 | none | yes | 6 | no |
| 56 | none | yes | 7 | no |
| 57 | GPS | no | 6 | no |
| 58 | GPS | no | 7 | no |
| 59 | GPS | yes | 6 | no |
| 60 | GPS | yes | 7 | no |

The films coated with a composition containing APS as the silane coupling agent (Comparative Examples Z, AA, AB and AC) fogged both with and without the addition of the wetting agent. The coated films which were applied using a number 6 Meyer bar (Comparative Examples Z and AB) appeared thin since the coatings had a gold hue, so a number 7 Meyer bar (Comparative Examples AA and AC) was used to get a thicker coating having a violet or blue hue. Regardless of the coating thickness, the coated films containing APS fogged. Unlike the coated films containing "Fluorad™ FC-95 Fluorochemical Surfactant" in Examples 50-52, coating thickness did not effect the anti-fog property. "Fluorad™ FC-120 Fluorochemical Surfactant" is an ammonium salt of a perfluoroaliphatic sulfonate. While not being bound to theory, in the presence of an organic amine base, such as APS, an ionic exchange could occur especially on heating which would drive off ammonia and form the APS salt of the perfluorinated sulfonate. The APS salt did not appear to be an effective anti-fog agent. In the absence of APS or with the coupling agent GPS, "Fluorad™ FC-120 Fluorochemical Surfactant" was a very effective anti-fog agent with or without the addition of wetting agent (see Examples 53-60). "Triton™ X-100" did not help prevent fogging and was not an effective anti-fog surfactant as shown in Comparative Example A after 12 days.

### Examples 61-63

### Comparative Examples AD-AH

Coating composition was prepared as described in Examples 43-49. Identical coating compositions were also prepared, except that the silane coupling agent, APS, was omitted (Examples 61-63 and Comparative Example AH). The surfactants used in the compositions are described in Table 18. The wetting agent if used was "Triton™ X-100." The coating compositions were applied to both sides of an unprimed flame treated 0.1 mm thick PET film using a number 7 wire wound rod Meyer bar. The coatings were dried and the fogging evaluated as described in Examples 43-49. The results of the fog test are given in Table 18.

**TABLE 18**

| **Example Number** | **Surfactant** | **Silane Coupling Agent** | **Wetting Agent** | **Fog** |
|---|---|---|---|---|
| Comp. AD | C₄F₉SO₃H | APS | no | yes |
| Comp. AE | FC-93 | APS | no | yes |
| Comp. AF | C₄F₉SO₃H | APS | yes | yes |
| Comp. AG | FC-93 | APS | yes | yes |
| 61 | C₄F₉SO₃H | none | no | no |
| 62 | FC-93 | none | no | no |
| Comp. AH | C₄F₉SO₃H | none | yes | yes |
| 63 | FC-93 | none | yes | no |

The films coated with a composition containing either surfactant, C₄F₉SO₃H or "Fluorad™ FC-93 Fluorochemical Surfactant," and APS as the silane coupling agent fogged both with and without the addition of the wetting agent (Comparative Examples AD, AE, AF and AG). FC-93 is an ammonium salt of a perfluoroaliphatic sulfonate. C₄F₉SO₃H is a very strong acid. These results were consistent with those for the "Fluorad™ FC-120 Fluorochemical Surfactant" from 3M Company in Examples 53-60 and Comparative Examples Z-AC and seem to indicate that in the presence of an organic base, such as APS, an ionic exchange occurs forming the APS salt of the perfluorinated sulfonate. The salt did not appear to be an effective anti-fog agent. However, in the absence of an additional amine (APS) both C₄F₉SO₃H and the "Fluorad™ FC-93 Fluorochemical Surfactant" from 3M Company were very effective anti-fog agents.

The coatings containing surfactant, C₄F₉SO₃H or "Fluorad™ FC-93 Fluorochemical Surfactant," without the silane coupling agent or the wetting agent (Examples 61 and 62) did not fog. Addition of the wetting agent to the coating composition containing C₄F₉SO₃H (Comparative Example AH) caused the film to fog while addition of the same wetting agent to the coating composition containing "Fluorad™ FC-93 Fluorochemical Surfactant" did not result in fogging.

## Claims

1. A coaling composition which imparts anti-reflection and anti-fogging properties to a substrate having at least one surface coated therewith, the coating composition comprising :
(a) an inorganic metal oxide sol capable of forming a porous inorganic metal oxide network which provides anti-reflective properties to a substrate ; and characterised in that the coating composition also comprises
(b) a surfactant having a solubility in water of less than 10 percent by weight at 23°C and comprised of at least one hydrophobic group and at least one hydrophilic anionic group, wherein:
(i) the hydrophilic anionic group comprises an anion selected from, the group consisting of -OSO₂O⁻, -SO₂O⁻, -CO₂⁻, (-O)₂P(O)O⁻, -OP(O)(O⁻)₂, -P(O)(O⁻)₂, -P(O⁻)₂, -OP(O⁻)₂, (-SO₂)₂N⁻, -SO₂N(R)⁻, (-SO₂)₂C⁻H, and -N⁺(R)₂(CH₂)ₓL', wherein R is hydrogen, an alkyl group which is unsubstituted or substituted with atoms independently selected from the group consisting of oxygen, nitrogen and sulfur, or an alkylene carboxyl group, which alkyl or alkylene carboxyl group comprises about 1 to 10 carbon atoms; x is 1 to 4; and L' is selected from the group consisting of -OSO₂O⁻, -SO₂O⁻, (-O)₂P(O)O⁻, -OP(O)(O⁻)₂, -P(O)(O⁻)₂ and -CO₂⁻; and wherein each anionic group is associated with or covalently bound to at least one cation, which cation is selected from the group consisting of H⁺, Na⁺, K⁺, Li⁺, Ca⁺², Mg⁺², Sr⁺², Al⁺³, and R"A⁺, wherein R" is R or R' wherein R is hydrogen or an alkyl or cycloalkyl group of about 1 to 10 carbon atoms, and R' is covalently bonded to the surfactant molecule and is an alkyl bridging group of 1 to 10 carbon atoms, and A is NR₃, a guanidinium ion optionally substituted with oxygen, nitrogen or sulfur atoms, or N⁺B wherein B comprises 3 to 7 atoms selected from the group consisting of carbon, nitrogen, sulfur and oxygen atoms which complete the nitrogen containing heterocyclic ring; and wherein any R or R' group may be unsubstituted or substituted with atoms independently selected from the group consisting of oxygen, nitrogen or sulfur; the cation selected such that the net charge of the surfactant molecule is neutral; and
(ii) wherein the hydrophobic group comprises a hydrocarbon chain comprising at least 4 carbon atoms, or a perfluorinated radical comprising at least 3 carbon atoms;
wherein the coating composition when coated on at least one side of a light transmissive substrate and dried provides a coated substrate with
(1) a porous inorganic metal oxide network of uniform average thickness;
(2) a drop diameter of at least about 4 mm when tested in accordance with the Wetting Test described herein; and
(3) a percent transmission at 550 nm which is at least 3 percent greater than that of the uncoated substrate.

2. The coating composition of claim 1 further characterised in that the surfactant has the general formula:
[(R)ₐL^{-c}]_{d}(M^{+b})ₑ
wherein :
R is selected from the group consisting of a perfluorinated alkyl or cycloalkyl group of about 3 to 18 perfluorinated carbon atoms; a polyethoxylated perfluoroalkyl or perfluorocycloalkyl substituted alcohol comprising about 3 to 18 perfluorinated carbon atoms and about 0 to 30 non-fluorinated carbon atoms; a perfluoroalkyl substituted alkyl or alkenyl group of about 3 to 18 perfluorinated carbon atoms and about 0 to 30 non-fluorinated carbon atoms, which alkyl or alkenyl group is unsubstituted or comprises oxygen, nitrogen or sulfur atoms within or substituted upon the alkyl or alkenyl chain; an alkyl or alkenyl group of about 4 to 36 carbon atoms, which alkyl or alkenyl is unsubstituted or comprises oxygen, nitrogen or sulfur atoms within or substituted upon the alkyl or alkenyl chain; an aralkyl group of about 7 to 36 carbon atoms, which aralkyl group is unsubstituted or independently substituted in available positions by oxygen, nitrogen or sulfur atoms; and a polyethoxylated or polypropoxylated alkyl or aralkyl group of about 7 to 36 carbon atoms;
L is selected from the group consisting of a sulfate, sulfonate, phosphate, phosphonate, sulfonimide, sulfonamide, carboxylate, phosphonite, phosphite and disulfonylmethide groups, and amphoteric alkyl forms thereof; provided that when L is a carboxylate, the surfactant molecule further comprises an additional polar heteroatom or substitutent no further than four atoms removed from the carboxylate group, wherein said polar substituent is selected from the group consisting of ether, amide, alcohol, carboxyl, ester, thioester, urea, and urethane groups, and combinations thereof;
M is selected from the group consisting of hydrogen, sodium, potassium, lithium, ammonium, calcium, magnesium, strontium, aluminum and R"A⁺, wherein R" is Ror R', wherein R is hydrogen or an alkyl or cycloalkyl group of about 1 to 10 carbon atoms and R' is covalently bonded to the surfactant molecule and is an alkyl bridging group of about 1 to 10 carbon atoms which is unsubstituted or substituted in available positions with oxygen, nitrogen or sulfur atoms; and A⁺ is selected from the group consisting of N⁺R₃; a guanidinium ion optionally substituted with oxygen, nitrogen or sulfur atoms; or a heterocyclic N⁺B wherein B comprises 3 to 7 atoms selected from the group consisting of carbon, nitrogen, sulfur and oxygen atoms which complete the nitrogen containing heterocyclic ring;
a and c are independently 1 or 2;
b and c are independently 1, 2 or 3; and
e is equal to (c times d)/b, or 0.

3. The coating composition of claim 1 further characterized in that the surfactant is a lithium, sodium or potassium salt of an anionic perfluoro-containing compound.

4. The coating composition of claim 1 further characterized in that the composition comprises between about 1 and about 5 percent by weight of the inorganic metal oxide particles and less than 0.15 percent by weight of the surfactant.

5. The coating composition of claim 1 further characterized in that the inorganic metal oxide is selected from the group consisting aluminum oxide, tin oxide, titanium oxide, antimony oxide, silica, zirconium oxide and mixtures thereof.

6. A coating composition which imparts anti-reflection and anti-fogging properties to a substrate having at least one surface coated therewith, the coating composition comprising :
(a) an inorganic metal oxide sol capable of forming a porous inorganic metal oxide network which provides anti-reflective properties to a substrate; and characterised in that said coating composition further comprises
(b) a surfactant having a solubility in water of less than 10 percent by weight at 23°C and comprised of at least one hydrophobic group and at least one hydrophilic anoinic group, wherein :
(i) the hydrophilic anionic group comprises an anion selected from the group consisting of -OSO₂O⁻, -SO₂O⁻, -CO₂⁻, (-O)₂P(O)O⁻, -OP(O)(O⁻)₂, -P(O)(O⁻)₂, -P(O⁻)₂, -OP(O⁻)₂, (-SO₂)₂N⁻, -SO₂N(R)⁻, (-SO₂)₂C⁻H, and -N⁺(R)₂(CH₂)ₓL', wherein R is hydrogen, an alkyl group which is unsubstituted of substituted with atoms independently selected from the group consisting of oxygen, nitrogen and sulfur, or an alkylene carboxyl group, which alkyl or alkylene carbonyl group comprises about 1 to 10 carbon atoms; x is 1 to 4; and L' is selected from the group consisting of -OSO₂O⁻, -SO₂O⁻, (-O)₂P(O)O⁻, -OP(O)(O⁻)₂, -P(O)(O⁻)₂ and -CO⁻₂; and wherein each anionic group is associated with or covalently bound to at least one cation, which cation is selected from the group consisting of H⁺, Na⁺, K⁺, Li⁺, Ca⁺², Mg⁺², Sr⁺², Al⁺³, and R"A⁺, wherein R" is R or R' wherein R is hydrogen or an alkyl or cycloalkyl group of about 1 to 10 carbon atoms, and R' is covalently bonded to the surfactant molecule and is an alkyl bridging group of 1 to 10 carbon atoms, and A⁺ is N⁺R₃, a guanidinium ion optioanlly substituted with oxygen, nitrogen or sulfur atoms, or N⁺B wherein B comprises 3 to 7 atoms selected from the group consisting of carbon, nitrogen, sulfur and oxygen atoms which complete the nitrogen containing heterocyclic ring; and wherein any R or R' group may be unsubstituted or substituted with atoms independently selected from the group consisting of oxygen, nitrogen or sulfur; the cation selected such that the net charge of the surfactant molecule is neutral; and
(ii) wherein the hydrophobic group comprises a hydrocarbon chain comprising at least 4 carbon atoms, or a perfluororinated radical comprising at least 3 carbon atoms; and wherein the surfactant optionally has at least one of the following properties :
(1) a melting point greater than 20°C; or
(2) is capable of being covalently bonded to metal oxide;
the surfactant being present in amounts necessary to provide anti-fogging properties to substrates coated on at least one surface with the coating composition and having a porous inorganic metal oxide network of uniform average thickness, but not sufficient to reduce the anti-reflection properties provided by the inorganic metal oxide to less than 3% greater than the uncoated substrate, measured by percent transmission of 550 nm light.

7. An article comprising a substrate having a surface and a layer of a coating composition according to claim 1 on the surface of at least one substrate which coating composition has been dried.

8. An article according to claim 7 further characterized in that the layer of the coating composition as a thickness in the range of about 500 to 2500Å.

9. An eye shield comprising a substrate which is transparent or translucent to visible light coated with
(a) a porous inorganic metal oxide network of uniform average thickness which provides anti-reflection properties to the substrate; and characterized in that said substrate is also coated with
(b) a surfactant having a solubility in water of less than 10 percent by weight at 23°C and comprised of at least one hydrophobic group and at least one hydrophilic anionic group, wherein:
(i) the hydrophilic anionic group comprises an anion selected from the group consisting of -OSO₂O⁻, -SO₂O⁻, -CO₂⁻, (-O)₂P(O)O⁻, -OP(O)(O⁻)₂, -P(O)(O⁻)₂, -P(O⁻)₂, -OP(O⁻)₂, (-SO₂)₂N⁻, -SO₂N(R)⁻, (-SO₂)₂C⁻H, and -N⁺(R)₂(CH₂)ₓL', wherein R is hydrogen, an alkyl group which is unsubstituted or substituted with atoms independently selected from the group consisting of oxygen, nitrogen and sulfur, or an alkylene carboxyl group, which alkyl or alkylene carboxyl group comprises about 1 to 10 carbon atoms; x is 1 to 4; and L' is selected from the group consisting of -OSO₂O⁻, -SO₂O⁻, (-O)₂P(O)O⁻, -OP(O)(O⁻)₂, -P(O)(O⁻)₂ and -CO₂⁻; and wherein each anionic group is associated with or covalently bound to at least one cation, which cation is selected from the group consisting of H⁺, Na⁺, K⁺, Li⁺, Ca⁺², Mg⁺², Sr⁺², Al⁺³, and R"A⁺, wherein R" is R or R' wherein R is hydrogen or an alkyl or cycloalkyl group of about 1 to 10 carbon atoms, and R' is covalently bonded to the surfactant molecule and is an alkyl bridging group of 1 to 10 carbon atoms, and A⁺ is N⁺R₃, a guanidinium ion optionally substituted with oxygen, nitrogen or sulfur atoms, or N⁺B wherein B comprises 3 to 7 atoms selected from the group consisting of carbon, nitrogen, sulfur and oxygen atoms which complete the nitrogen containing heterocyclic ring; and wherein any R or R' group may be unsubstituted or substituted with atoms independently selected from the group consisting of oxygen, nitrogen or sulfur; the cation selected such that the net charge of the surfactant molecule is neutral; and
(ii) wherein the hydrophobic group comprises a hydrocarbon chain comprising at least 4 carbon atoms, or a perfluorinated radical comprising at least 3 carbon atoms;
which coated substrate when coated on at least one surface exhibits
1) a drop diameter of at least 4 mm when tested in accordance with the Wetting Test described herein; and
2) a percent transmission at 550 nm which is at least 3 percent greater than that of the uncoated substrate.

10. The eye shield of claim 9 further characterized in that the inorganic metal oxide is silica and the surfactant is a perfluorinated sulfonate.

11. A surgical mask comprising a face mask and an eye shield according to claim 9.

12. A method of imparting anti-reflection and anti-fogging properties to a substrate, the method comprising the steps of:
(a) providing a substrate;
(b) preparing a coating composition;
(c) applying the coating composition to at least one side of the substrate; and
(d) drying the coating composition; characterized in that the coating composition is the coating composition of anyone of claim 1 to 6 to produce a coated substrate having a porous inorganic metal oxide network of uniform average thickness.

13. A method of imparting anti-reflection and anti-fogging properties to a substrate, the method comprising the steps of:
(a) providing a substrate;
(b) preparing a first coating composition comprising an inorganic metal oxide sol capable of forming a porous inorganic metal oxide network which provides anti-reflective properties to a substrate ;
(c) preparing a second coating composition;
(d) applying either the first or second coating composition to at least one side of the substrate;
(e) allowing the coating applied in step (d) to dry;
(f) applying the coating composition not applied in step (d) to at least one side of the substrate;
(g) allowing the coating applied in step (f) to dry; characterized in that the second coating composition comprises a surfactant having a solubility in water of less than 10 percent by weight at 23°C and having at least one hydrophobic group and at least one hydrophilic anionic group, wherein :
(i) the hydrophilic anionic group comprises an anion selected from the group consisting of -OSO₂O⁻, -SO₂O⁻, -CO₂⁻, (-O)₂P(O)O⁻, -OP(O)(O⁻)₂, -P(O)(O⁻)₂, -P(O⁻)₂, -OP(O⁻)₂, (-SO₂)₂N⁻, -SO₂N(R)⁻, (-SO₂)₂C⁻H, and -N⁺(R)₂(CH₂)ₓL', wherein R is hydrogen, an alkyl group which is unsubstituted or substituted with atoms independently selected from the group consisting of oxygen, nitrogen and sulfur, or an alkylene carboxyl group, which alkyl or alkylene carboxyl group comprises about 1 to 10 carbon atoms; x is 1 to 4; and L' is selected from the group consisting of -OSO₂O⁻, -SO₂O⁻, the group consisting of -OSO₂O⁻, -SO₂O⁻, (-O)₂P(O)O⁻, -OP(O)(O⁻)₂, -P(O)(O⁻)₂ and -CO₂⁻; and wherein each anionic group is associated with or covalently bound to at least one cation, which cation is selected from the group consisting of H⁺, Na⁺, K⁺, Li⁺, Ca⁺², Mg⁺², Sr⁺², Al⁺³, and R"A⁺, wherein R" is R or R' wherein is hydrogen or an alkyl or cycloalkyl group of about 1 to 10 carbon atoms, and R' is covalently bonded to the surfactant molecule and is an alkyl bridging group of 1 to 10 carbon atoms, and A⁺ is N⁺R₃, a guanidinium ion optionally substituted with oxygen, nitrogen or sulfur atoms, or N⁺B wherein B comprises 3 to 7 atoms selected from the group consisting of carbon, nitrogen, sulfur and oxygen atoms which complete the nitrogen containing heterocyclic ring; and wherein any R or R' group may be unsubstituted or substituted with atoms independently selected from the group consisting of oxygen, nitrogen or sulfur; the cation selected such that the net charge of the surfactant molecule is neutral; and
(ii) wherein the hydrophobic group comprises a hydrocarbon chain comprising at least 4 carbon atoms, or a perfluorinated radical comprising at least 3 carbon atoms.

## Patentansprüche

1. Beschichtungszusammensetzung, die einem Substrat mit mindestens einer damit beschichteten Oberfläche Antireflexions- und Antibeschlagseigenschaften vermittelt, wobei die Beschichtungszusammensetzung umfaßt:
(a) ein Sol eines anorganischen Metalloxids, das imstande ist, ein poröses Netzwerk aus anorganischem Metalloxid, das reflexmindernde Eigenschaften für das Substrat bereitstellt, zu erzeugen, und dadurch gekennzeichnet, daß die Beschichtungszusammensetzung außerdem umfaßt
(b) einen grenzflächenaktiven Stoff mit einer Löslichkeit in Wasser von weniger als 10 Gewichtsprozent bei 23°C und umfassend mindestens einen hydrophoben Rest und mindestens einen hydrophilen anionischen Rest, wobei:
(i) der hydrophile anionische Rest ein Anion umfaßt, ausgewählt aus - OSO₂O⁻, -SO₂O⁻, -CO₂⁻, (-O)₂P(O)O⁻, -OP(O)(O⁻)₂, -P(O)(O⁻)₂, -P(O⁻)₂, -OP(O⁻)₂, (-SO₂)₂N⁻, -SO₂N(R)⁻, (-SO₂)₂C⁻H und -N⁺(R)₂(CH₂)ₓL', wobei R ein Wasserstoffatom, ein Alkylrest, der unsubstituiert oder mit Atomen, unabhängig ausgewählt aus Sauerstoff-, Stickstoff- und Schwefelatomen, substituiert ist, oder ein Alkylencarboxylrest ist, und dieser Alkyl- oder Alkylencarboxylrest etwa 1 bis 10 Kohlenstoffatome umfaßt; x 1 bis 4 ist; und L' aus -OSO₂O⁻, -SO₂O⁻, (-O)₂P(O)O⁻, -OP(O)(O⁻)₂, -P(O)(O⁻)₂ und -CO₂⁻ ausgewählt ist; und wobei jeder anionische Rest assoziiert mit oder kovalent gebunden an mindestens ein Kation ist, und dieses Kation aus H⁺, Na⁺, K⁺, Li⁺, Ca²⁺, Mg²⁺, Sr²⁺, Al³⁺ und R"A⁺ ausgewählt ist, wobei R'' R oder R' ist, wobei R ein Wasserstoffatom oder ein Alkyl- oder Cycloalkylrest mit etwa 1 bis 10 Kohlenstoffatomen ist und R' kovalent an das grenzflächenaktive Molekül gebunden ist und ein Alkylbrückenrest mit 1 bis 10 Kohlenstoffatomen ist, und A NR₃, ein Guanidiniumion, gegebenenfalls substituiert mit Sauerstoff-, Stickstoff- oder Schwefelatomen, oder N⁺B ist, wobei B 3 bis 7 Atome, ausgewählt aus Kohlenstoff-, Stickstoff-, Schwefel- und Sauerstoffatomen, umfaßt, die den Stickstoff enthaltenden heterocyclischen Ring vervollständigen; und wobei jeder Rest R oder R' unsubstituiert oder mit Atomen, unabhängig ausgewählt aus Sauerstoff-, Stickstoff- oder Schwefelatomen, substituiert sein kann; das Kation derart ausgewählt ist, daß die Nettoladung des grenzflächenaktiven Moleküls neutral ist; und
(ii) wobei der hydrophobe Rest eine Kohlenwasserstoffkette, umfassend mindestens 4 Kohlenstoffatome, oder einen perfluorierten Rest, umfassend mindestens 3 Kohlenstoffatome, umfaßt;
wobei die Beschichtungszusammensetzung, wenn auf mindestens eine Seite eines lichtdurchlässigen Substrats aufgebracht und getrocknet, ein beschichtetes Substrat mit:
(1) einem porösen Netzwerk aus anorganischem Metalloxid gleichmäßiger durchschnittlicher Dicke;
(2) einem Tropfendurchmesser von mindestens etwa 4 mm, wenn gemäß dem hierin beschriebenen Benetzungstest getestet; und
(3) einer prozentualen Durchlässigkeit bei 550 nm, die mindestens 3 Prozent größer als die des unbeschichteten Substrats ist;
bereitstellt.

2. Beschichtungszusammensetzung nach Anspruch 1, weiterhin dadurch gekennzeichnet, daß der grenzflächenaktive Stoff die allgemeine Formel hat:
[(R)ₐL^{-c}]_{d}(M^{+b})ₑ
wobei:
R aus einem perfluorierten Alkyl- oder Cycloalkylrest mit etwa 3 bis 18 perfluorierten Kohlenstoffatomen; einem polyethoxylierten, mit einem Perfluoralkyl- oder Perfluorcycloalkylrest substituierten Alkohol, umfassend etwa 3 bis 18 perfluorierte Kohlenstoffatome und etwa 0 bis 30 nichtfluorierte Kohlenstoffatome; einem mit einem Perfluoralkylrest substituierten Alkyl- oder Alkenylrest mit etwa 3 bis 18 perfluorierten Atomen und etwa 0 bis 30 nichtfluorierten Kohlenstoffatomen, und dieser Alkyl- oder Alkenylrest unsubstituiert ist oder Sauerstoff-, Stickstoff- oder Schwefelatome innerhalb oder substituiert auf der Alkyl- oder Alkenylkette umfaßt; einem Alkyl- oder Alkenylrest mit etwa 4 bis 36 Kohlenstoffatomen, und dieser Alkyl- oder Alkenylrest unsubstituiert ist oder Sauerstoff-, Stickstoff- oder Schwefelatome innerhalb oder substituiert auf der Alkyl- oder Alkenylkette umfaßt; einem Aralkylrest mit etwa 7 bis 36 Kohlenstoffatomen, und dieser Aralkylrest unsubstituiert oder in verfügbaren Positionen durch Sauerstoff-, Stickstoff- oder Schwefelatome unabhängig substituiert ist; und einem polyethoxylierten oder polypropoxylierten Alkyl- oder Aralkylrest mit etwa 7 bis 36 Kohlenstoffatomen ausgewählt ist;
L aus einer Sulfat-, Sulfonat-, Phosphat-, Phosphonat-, Sulfonimid-, Sulfonamid-, Carboxylat-, Phosphonit-, Phosphit- und Disulfonylmethidgruppe sowie amphoteren Alkylformen davon ausgewählt ist; mit der Maßgabe, daß, wenn L ein Carboxylat ist, das grenzflächenaktive Molekül weiterhin ein zusätzliches polares Heteroatom oder einen Substituenten umfaßt, der nicht weiter als vier Atome von der Carboxylatgruppe entfernt ist, wobei der polare Substituent aus Ether-, Amid-, Alkohol-, Carboxyl-, Ester-, Thioester-, Harnstoff- und Urethanresten sowie Kombinationen davon ausgewählt ist;
M aus Wasserstoff, Natrium, Kalium, Lithium, Ammonium, Calcium, Magnesium, Strontium, Aluminium und R"A⁺ ausgewählt ist, wobei R" R oder R' ist, wobei R ein Wasserstoffatom oder ein Alkyl- oder Cycloalkylrest mit etwa 1 bis 10 Kohlenstoffatomen ist und R' kovalent an das grenzflächenaktive Molekül gebunden ist und ein Alkylbrückenrest mit etwa 1 bis 10 Kohlenstoffatomen ist, der unsubstituiert oder in verfügbaren Positionen mit Sauerstoff-, Stickstoff- oder Schwefelatomen substituiert ist; und A⁺ aus N⁺R₃; einem Guanidiniumion, gegebenenfalls substituiert mit Sauerstoff-, Stickstoff- oder Schwefelatomen; oder einem heterocyclischen N⁺B, wobei B 3 bis 7 Atome, ausgewählt aus Kohlenstoff-, Stickstoff-, Schwefel- und Sauerstoffatomen, umfaßt, die den Stickstoff enthaltenden heterocyclischen Ring vervollständigen, ausgewählt ist;
a und c unabhängig 1 oder 2 sind;
b und d unabhängig 1, 2 oder 3 sind; und
e gleich (c mal d)/b oder 0 ist.

3. Beschichtungszusammensetzung nach Anspruch 1, weiterhin dadurch gekennzeichnet, daß der grenzflächenaktive Stoff ein Lithium-, Natrium- oder Kaliumsalz einer anionischen perfluorhaltigen Verbindung ist.

4. Beschichtungszusammensetzung nach Anspruch 1, weiterhin dadurch gekennzeichnet, daß die Zusammensetzung zwischen etwa 1 und etwa 5 Gewichtsprozent der Teilchen des anorganischen Metalloxids und weniger als 0,15 Gewichtsprozent des grenzflächenaktiven Stoffes umfaßt.

5. Beschichtungszusammensetzung nach Anspruch 1, weiterhin dadurch gekennzeichnet, daß das anorganische Metalloxid aus Aluminiumoxid, Zinnoxid, Titanoxid. Antimonoxid, Siliciumdioxid, Zirconiumoxid und Gemischen davon ausgewählt ist.

6. Beschichtungszusammensetzung, die einem Substrat mit mindestens einer damit beschichteten Oberfläche Antireflexions- und Antibeschlagseigenschaften vermittelt, wobei die Beschichtungszusammensetzung umfaßt:
(a) ein Sol eines anorganischen Metalloxids, das imstande ist, ein poröses Netzwerk aus anorganischem Metalloxid, das reflexmindernde Eigenschaften für ein Substrat bereitstellt, zu erzeugen, und dadurch gekennzeichnet, daß die Beschichtungszusammensetzung weiterhin umfaßt
(b) einen grenzflächenaktiven Stoff mit einer Löslichkeit in Wasser von weniger als 10 Gewichtsprozent bei 23°C und bestehend aus mindestens einem hydrophoben Rest und mindestens einem hydrophilen anionischen Rest, wobei:
(i) der hydrophile anionische Rest ein Anion umfaßt, ausgewählt aus -OSO₂O⁻, -SO₂O⁻, -CO₂⁻, (-O)₂P(O)O⁻, -OP(O)(O⁻)₂, -P(O)(O⁻)₂, -P(O)₂, -OP(O⁻)₂, (-SO₂)₂N⁻, -SO₂N(R)⁻, (-SO₂)₂C⁻H und -N⁺(R)₂(CH₂)ₓL', wobei R ein Wasserstoffatom, ein Alkylrest, der unsubstituiert oder mit Atomen, unabhängig ausgewählt aus Sauerstoff-, Stickstoff- und Schwefelatomen, substituiert ist, oder ein Alkylencarboxylrest ist, und dieser Alkyl- oder Alkylencarboxylrest etwa 1 bis 10 Kohlenstoffatome umfaßt; x 1 bis 4 ist; und L' aus -OSO₂O⁻, -SO₂O⁻, (-O)₂P(O)O⁻, -OP(O)(O⁻)₂, -P(O)(O⁻)₂ und -CO₂⁻ ausgewählt ist; und wobei jeder anionische Rest assoziiert mit oder kovalent gebunden ist an mindestens ein Kation, und dieses Kation aus H⁺, Na⁺, K⁺, Li⁺, Ca²⁺, Mg²⁺, Sr²⁺, Al³⁺ und R"A⁺ ausgewählt ist, wobei R'' R oder R' ist, wobei R ein Wasserstoffatom oder ein Alkyl- oder Cycloalkylrest mit etwa 1 bis 10 Kohlenstoffatomen ist und R' kovalent an das grenzflächenaktive Molekül gebunden ist und ein Alkylbrückenrest mit 1 bis 10 Kohlenstoffatomen ist, und A⁺ aus N⁺R₃, ein Guanidiniumion, gegebenenfalls substituiert mit Sauerstoff-, Stickstoff- oder Schwefelatomen, oder N⁺B ist, wobei B 3 bis 7 Atome umfaßt, ausgewählt aus Kohlenstoff-, Stickstoff-, Schwefel- und Sauerstoffatomen, die den Stickstoff enthaltenden heterocyclischen Ring vervollständigen; und wobei jeder Rest R oder R' unsubstituiert oder mit Atomen, unabhängig ausgewählt aus Sauerstoff-, Stickstoff- und Schwefelatomen, substituiert sein kann; das Kation derart ausgewählt ist, daß die Nettoladung des grenzflächenaktiven Moleküls neutral ist; und
(ii) wobei der hydrophobe Rest eine Kohlenwasserstoffkette, umfassend mindestens 4 Kohlenstoffatome, oder einen perfluorierten Rest, umfassend mindestens 3 Kohlenstoffatome, umfaßt; und wobei der grenzflächenaktive Stoff gegebenenfalls mindestens eine der folgenden Eigenschaften hat:
(1) einen Schmelzpunkt von mehr als 20°C; oder
(2) imstande ist, kovalent an Metalloxid gebunden zu werden;
wobei der grenzflächenaktive Stoff in Mengen vorhanden ist, die notwendig sind, um für Substrate, die auf mindestens einer Oberfläche mit der Beschichtungszusammensetzung beschichtet sind und die ein poröses Netzwerk aus anorganischem Metalloxid gleichmäßiger durchschnittlicher Dicke aufweisen, Beschlagverhinderungseigenschaften bereitzustellen, aber nicht ausreichend sind, um die Reflexionsminderungseigenschaften, bereitgestellt durch das anorganische Metalloxid, auf weniger als 3% mehr als das unbeschichtete Substrat, gemessen durch Prozent Durchlässigkeit für Licht von 550 nm, zu verringern.

7. Gegenstand, umfassend ein Substrat mit einer Oberfläche und eine Schicht einer Beschichtungszusammensetzung nach Anspruch 1 auf der Oberfläche mindestens eines Substrats, wobei diese Beschichtungszusammensetzung getrocknet worden ist.

8. Gegenstand nach Anspruch 7, weiterhin dadurch gekennzeichnet, daß die Schicht der Beschichtungszusammensetzung eine Dicke im Bereich von etwa 500 bis 2500 Å hat.

9. Augenschutz, umfassend ein Substrat, das für sichtbares Licht durchsichtig oder durchscheinend ist, beschichtet mit
(a) einem porösen Netzwerk aus anorganischem Metalloxid gleichmäßiger durchschnittlicher Dicke, das Reflexionsminderungseigenschaften für das Substrat bereitstellt; und dadurch gekennzeichnet, daß das Substrat außerdem beschichtet ist mit
(b) einem grenzflächenaktiven Stoff mit einer Löslichkeit in Wasser von weniger als 10 Gewichtsprozent bei 23°C und bestehend aus mindestens einem hydrophoben Rest und mindestens einem hydrophilen anionischen Rest, wobei:
(i) der hydrophile anionische Rest ein Anion umfaßt, ausgewählt aus -OSO₂O⁻, -SO₂O⁻, -CO₂⁻, (-O)₂P(O)O⁻, -OP(O)(O⁻)₂, -P(O)(O⁻)₂, -P(O⁻)₂, -OP(O⁻)₂, (-SO₂)₂N⁻, -SO₂N(R)⁻, (-SO₂)₂C⁻H und -N⁺(R)₂(CH₂)ₓL', wobei R ein Wasserstoffatom, ein Alkylrest, der unsubstituiert oder mit Atomen, unabhängig ausgewählt aus Sauerstoff-, Stickstoff- und Schwefelatomen, substituiert ist, oder ein Alkylencarboxylrest ist, und dieser Alkyl- oder Alkylencarboxylrest etwa 1 bis 10 Kohlenstoffatome umfaßt; x 1 bis 4 ist; und L' aus -OSO₂O⁻, -SO₂O⁻, (-O)₂P(O)O⁻, -OP(O)(O⁻)₂, -P(O)(O⁻)₂ und -CO₂⁻ ausgewählt ist; wobei jeder anionische Rest assoziiert mit oder kovalent gebunden an mindestens ein Kation ist, und dieses Kation aus H⁺, Na⁺, K⁺, Li⁺, Ca²⁺, Mg²⁺, Sr²⁺, Al³⁺ und R''A⁺ ausgewählt ist, wobei R'' R oder R' ist, wobei R ein Wasserstoffatom oder ein Alkyl- oder Cycloalkylrest mit etwa 1 bis 10 Kohlenstoffatomen ist und R' kovalent an das grenzflächenaktive Molekül gebunden ist und ein Alkylbrückenrest mit 1 bis 10 Kohlenstoffatomen ist, und A⁺ aus N⁺R₃, ein Guanidiniumion, gegebenenfalls substituiert mit Sauerstoff-, Stickstoff- oder Schwefelatomen, oder N⁺B ist, wobei B 3 bis 7 Atome, ausgewählt aus Kohlenstoff-, Stickstoff-, Schwefel- und Sauerstoffatomen, umfaßt, die den Stickstoff enthaltenden heterocyclischen Ring vervollständigen; und wobei jeder Rest R oder R' unsubstituiert oder mit Atomen, unabhängig ausgewählt aus Sauerstoff-, Stickstoff- oder Schwefelatomen, substituiert sein kann; das Kation derart ausgewählt ist, daß die Nettoladung des grenzflächenaktiven Moleküls neutral ist; und
(ii) wobei der hydrophobe Rest eine Kohlenwasserstoffkette, umfassend mindestens 4 Kohlenstoffatome, oder einen perfluorierten Rest, umfassend mindestens 3 Kohlenstoffatome, umfaßt;
und dieses beschichtete Substrat, wenn auf mindestens einer Oberfläche beschichtet,
(1) einen Tropfendurchmesser von mindestens 4 mm, wenn gemäß dem hierin beschriebenen Benetzungstest getestet; und
(2) eine prozentuale Durchlässigkeit bei 550 nm, die mindestens 3 Prozent größer als die des unbeschichteten Substrats ist;
zeigt.

10. Augenschutz nach Anspruch 9, weiterhin dadurch gekennzeichnet, daß das anorganische Metalloxid Siliciumdioxid ist und der grenzflächenaktive Stoff ein perfluoriertes Sulfonat ist.

11. Chirurgische Maske, umfassend eine Gesichtsmaske und einen Augenschutz nach Anspruch 9.

12. Verfahren zum Vermitteln von Antireflexions- und Antibeschlagseigenschaften an ein Substrat, umfassend die Schritte:
(a) Bereitstellen eines Substrats;
(b) Herstellen einer Beschichtungszusammensetzung;
(c) Aufbringen der Beschichtungszusammensetzung auf mindestens eine Seite des Substrats; und
(d) Trocknen der Beschichtungszusammensetzung; dadurch gekennzeichnet, daß die Beschichtungszusammensetzung die Beschichtungszusammensetzung nach einem der Ansprüche 1 bis 6 ist, um ein beschichtetes Substrat, das ein poröses Netzwerk aus anorganischem Metalloxid gleichmäßiger durchschnittlicher Dicke aufweist, herzustellen.

13. Verfahren zum Vermitteln von Antireflexions- und Antibeschlagseigenschaften an ein Substrat, umfassend die Schritte:
(a) Bereitstellen eines Substrats;
(b) Herstellen einer ersten Beschichtungszusammensetzung, umfassend ein Sol eines anorganischen Metalloxids, das imstande ist, ein poröses Netzwerk aus anorganischem Metalloxid, das reflexmindernde Eigenschaften für ein Substrat bereitstellt, zu erzeugen;
(c) Herstellen einer zweiten Beschichtungszusammensetzung;
(d) Aufbringen entweder der ersten oder der zweiten Beschichtungszusammensetzung auf mindestens eine Seite des Substrats; und
(e) Trocknenlassen der in Schritt (d) aufgebrachten Beschichtung;
(f) Aufbringen der in Schritt (d) nicht aufgebrachten Beschichtungszusammensetzung auf mindestens eine Seite des Substrats;
(g) Trocknenlassen der in Schritt (f) aufgebrachten Beschichtung; dadurch gekennzeichnet, daß die zweite Beschichtungszusammensetzung einen grenzflächenaktiven Stoff mit einer Löslichkeit in Wasser von weniger als 10 Gewichtsprozent bei 23°C und mit mindestens einem hydrophoben Rest und mit mindestens einem hydrophilen anionischen Rest umfaßt, wobei:
(i) der hydrophile anionische Rest ein Anion umfaßt, ausgewählt aus -OSO₂O⁻, -SO₂O⁻, -CO₂⁻, (-O)₂P(O)O⁻, -OP(O)(O⁻)₂, -P(O)(O⁻)₂, -P(O⁻)₂, -OP(O⁻)₂, (-SO₂)₂N⁻, -SO₂N(R)⁻, (-SO₂)₂C⁻H und -N⁺(R)₂(CH₂)ₓL', wobei R ein Wasserstoffatom, ein Alkylrest, der unsubstituiert oder mit Atomen, unabhängig ausgewählt aus Sauerstoff-, Stickstoff- und Schwefelatomen, substituiert ist, oder ein Alkylencarboxylrest ist, und dieser Alkyl- oder Alkylencarboxylrest etwa 1 bis 10 Kohlenstoffatome umfaßt; x 1 bis 4 ist; und L' aus -OSO₂O⁻, -SO₂O⁻, (-O)₂P(O)O⁻, -OP(O)(O⁻)₂, -P(O)(O⁻)₂ und -CO₂⁻ ausgewählt ist; und wobei jeder anionische Rest assoziiert mit oder kovalent gebunden an mindestens ein Kation ist, und dieses Kation aus H⁺, Na⁺, K⁺, Li⁺, Ca²⁺, Mg²⁺, Sr²⁺, Al³⁺ und R"A⁺ ausgewählt ist, wobei R'' R oder R' ist, wobei R ein Wasserstoffatom oder ein Alkyl- oder Cycloalkylrest mit etwa 1 bis 10 Kohlenstoffatomen ist und R' kovalent an das grenzflächenaktive Molekül gebunden ist und ein Alkylbrückenrest mit 1 bis 10 Kohlenstoffatomen ist, und A⁺ aus N⁺R₃, ein Guanidiniumion, gegebenenfalls substituiert mit Sauerstoff-, Stickstoff- oder Schwefelatomen, oder N⁺B ist, wobei B 3 bis 7 Atome, ausgewählt aus Kohlenstoff-, Stickstoff-, Schwefel- und Sauerstoffatomen, umfaßt, die den Stickstoff enthaltenden heterocyclischen Ring vervollständigen; und wobei jeder Rest R oder R' unsubstituiert oder mit Atomen, unabhängig ausgewählt aus Sauerstoff-, Stickstoff- oder Schwefelatomen, substituiert sein kann; das Kation derart ausgewählt ist, daß die Nettoladung des grenzflächenaktiven Moleküls neutral ist; und
(ii) wobei der hydrophobe Rest eine Kohlenwasserstoffkette, umfassend mindestens 4 Kohlenstoffatome, oder einen perfluorierten Rest. umfassend mindestens 3 Kohlenstoffatome, umfaßt.

## Revendications

1. Composition d'enduction qui communique des propriétés antireflet et anti-buée à un substrat ayant au moins une surface recouverte par celle-ci, la composition d'enduction comprenant :
(a) un sol d'oxyde métallique inorganique capable de former un réseau d'oxyde métallique inorganique poreux qui communique à un substrat des propriétés antireflet; et caractérisé en ce que la composition d'enduction comprend également
(b) un agent tensioactif ayant une solubilité dans l'eau de moins de 10 pour cent en poids à 23°C et qui comprend au moins un groupe hydrophobe et au moins un groupe anionique hydrophile, dans lequel
(i) le groupe hydrophile anionique comprend un anion choisi parmi -OSO₂O⁻, -SO₂O⁻, -CO₂⁻, (-O)₂P(O)O⁻, -OP(O)(O⁻)₂, -P(O)(O⁻)₂, -P(O⁻)₂, -OP(O⁻)₂, (-SO₂)₂N⁻, -SO₂N(R)⁻, (-SO₂)₂C⁻H et -N⁺(R)₂(CH₂)ₓL', dans lequel R est l'hydrogène, un groupe alkyle qui est non substitué ou substitué avec des atomes choisis indépendamment parmi l'oxygène, l'azote et le soufre, ou un groupe alkylène-carboxyle, lequel groupe alkyle ou alkylène carboxyle comprend environ de 1 à 10 atomes de carbone; x est 1 à 4; et L' est choisi parmi -OSO₂O⁻, -SO₂O⁻, (-O)₂P(O)O⁻, -OP(O)(O⁻)₂, -P(O)(O⁻)₂, et -CO⁻₂; et dans lequel chaque groupe anionique est associé ou lié par covalence avec au moins un cation, lequel cation est choisi parmi H⁺, Na⁺, K⁺, Li⁺, Ca²⁺, Mg²⁺, Sr²⁺, Al³⁺, et les groupes R"A⁺, dans lesquels R" est R ou R' dans lesquels R est l'hydrogène ou un groupe alkyle ou cycloalkyle ayant de 1 à 10 atomes de carbone, et R' est lié par covalence à la molécule d'agent tensioactif et est un groupe alkyle pontant de 1 à 10 atomes de carbone, et A est NR₃, un ion guanidinium éventuellement substitué avec des atomes d'oxygène, d'azote ou de soufre, ou N⁺B dans lequel B comprend de 3 à 7 atomes choisis parmi les atomes de carbone, d'azote, de soufre et d'oxygène qui complètent le noyau hétérocyclique contenant l'azote; et dans lequel un groupe quelconque R ou R' peut être non substitué ou substitué avec des atomes choisis indépendamment parmi l'oxygène, l'azote ou le soufre; le cation est choisi de telle sorte que la charge nette de la molécule d'agent tensioactif soit neutre; et
(ii) dans lequel le groupe hydrophobe comprend une chaîne hydrocarbonée renfermant au moins 4 atomes de carbone, ou un radical perfluoré renfermant au moins 3 atomes de carbone;
dans lequel la composition d'enduction, lorsqu'elle est déposée et séchée sur au moins une face d'un substrat transmettant la lumière permet d'obtenir un substrat revêtu possédant
(1) un réseau d'oxyde métallique inorganique poreux d'épaisseur moyenne uniforme;
(2) un diamètre de goutte d'au moins environ 4 mm à l'essai conforme au Test de Mouillage décrit ici; et
(3) un pourcentage de transmission à 550 nm qui est supérieur d'au moins 3 pour cent à celui du substrat non revêtu.

2. Composition d'enduction selon la revendication 1 caractérisée en outre en ce que l'agent tensioactif a la formule générale :
[(R)ₐL^{-c}]_{d}(M^{+b})ₑ
dans laquelle
R est choisi parmi un groupe alkyle ou cycloalkyle perfluoré ayant entre environ 3 et 18 atomes de carbone perfluorés; un alcool polyéthoxylé, substitué par un perfluoroalkyle ou un perfluoro-cycloalkyle, comprenant environ de 3 à 18 atomes de carbone perfluorés et environ de 0 à 30 atomes de carbone non fluorés; un groupe alkyle ou alcényle substitué par un perfluoroalkyle ayant entre environ de 3 à 18 atomes de carbone perfluorés et environ de 0 à 30 atomes de carbone non fluorés, lequel groupe alkyle ou alcényle est non substitué ou comprend des atomes d'oxygène, d'azote ou de soufre à l'intérieur de la chaîne alkyle ou alcényle ou substitués sur celle-ci; un groupe alkyle ou alcényle ayant entre environ 4 et 36 atomes de carbone, lequel groupe alkyle ou alcényle est non substitué ou comprend des atomes d'oxygène, d'azote ou de soufre à l'intérieur de la chaîne alkyle ou alcényle ou substitués sur celle-ci; un groupe aralkyle ayant entre environ 7 et 36 atomes de carbone, lequel groupe aralkyle est non substitué ou indépendamment, substitué dans des positions disponibles par des atomes d'oxygène, d'azote ou de soufre; et un groupe alkyle ou aralkyle polyéthoxylé ou polypropoxylé ayant entre environ 7 et 36 atomes de carbone;
L est choisi parmi les groupes sulfate, sulfonate, phosphate, phosphonate, sulfonimide, sulfonamide, carboxylate, phosphonite, phosphite et disulfonyl-méthanure, et les formes alkyliques amphotères de ceux-ci; à condition que lorsque L est un carboxylate, et que la molécule d'agent tensioactif comprend en outre un hétéroatome ou un substituant polaire additionnel, pas plus de quatre atomes ne soient enlevés du groupe carboxylate, dans lequel ledit substituant polaire est choisi parmi les groupes éther, amide, alcool, carboxyle, ester, thioester, urée, et uréthane, et les combinaisons de ceux-ci;
M est choisi parmi l'hydrogène, le sodium, le potassium, le lithium, l'ammonium, le calcium, le magnésium, le strontium, l'aluminium et R"A⁺ dans laquelle R" est R ou R', dans laquelle R est un atome d'hydrogène ou un groupe alkyle ou cycloalkyle ayant environ de 1 à 10 atomes de carbone et R' est lié par covalence à la molécule d'agent tensioactif et est un groupe alkyle pontant ayant environ de 1 à 10 atomes de carbone qui est non substitué ou substitué dans des positions disponibles par des atomes d'oxygène, d'azote ou de soufre; et
A⁺ est choisi parmi N⁺R₃, un ion guanidinium éventuellement substitué avec des atomes d'oxygène, d'azote ou de soufre, ou un groupe hétérocyclique N⁺B dans lequel B comprend de 3 à 7 atomes choisis parmi les atomes de carbone, d'azote, de soufre et d'oxygène qui complètent le noyau hétérocyclique contenant l'azote;
a et c sont indépendamment 1 ou 2;
b et d sont indépendamment 1, 2 ou 3; et
e est égal à (c fois d)/b, ou 0.

3. Composition d'enduction selon la revendication 1 caractérisée en outre en ce que l'agent tensioactif est un sel de lithium, de sodium ou de potassium d'un composé anionique perfluoré.

4. Composition d'enduction selon la revendication 1 caractérisée en outre en ce que la composition comprend entre environ 1 et environ 5 pour cent des particules d'oxyde métallique inorganique et moins de 0,15 pour cent en poids de l'agent tensioactif.

5. Composition d'enduction selon la revendication 1 caractérisée en outre en ce que l'oxyde métallique inorganique est choisi parmi l'oxyde d'aluminium, l'oxyde d'étain, l'oxyde de titane, l'oxyde d'antimoine, la silice, l'oxyde de zirconium et les mélanges de ceux-ci.

6. Composition d'enduction qui communique des propriétés antireflet et anti-buée à un substrat ayant au moins une surface recouverte par celle-ci, la composition d'enduction comprenant :
(a) un sol d'oxyde métallique inorganique capable de former un réseau d'oxyde métallique inorganique poreux qui communique à un substrat des propriétés antireflet; et caractérisé en ce que ladite composition d'enduction comprend également
(b) un agent tensioactif ayant une solubilité dans l'eau de moins de 10 pour cent en poids à 23°C et qui comprend au moins un groupe hydrophobe et au moins un groupe anionique hydrophile, dans lequel :
(i) le groupe hydrophile anionique comprend un anion choisi parmi -OSO₂O⁻, -SO₂O⁻, -CO₂⁻, (-O)₂P(O)O⁻, -OP(O)(O⁻)₂, -P(O)(O⁻)₂, -P(O⁻)₂, -OP(O⁻)₂, (-SO₂)₂N⁻, -SO₂N(R)⁻, (-SO₂)₂C⁻H et -N⁺(R)₂(CH₂)ₓL', dans lequel R est l'hydrogène, un groupe alkyle qui est non substitué ou substitué avec des atomes choisis indépendamment parmi l'oxygène, l'azote et le soufre, ou un groupe alkylène-carboxyle, lequel groupe alkyle ou alkylène carboxyle comprend environ de 1 à 10 atomes de carbone; x est 1 à 4; et L' est choisi parmi -OSO₂O⁻, -SO₂O⁻, (-O)₂P(O)O⁻, -OP(O)(O⁻)₂, -P(O)(O⁻)₂, et -CO⁻_{2;} et dans lequel chaque groupe anionique est associé ou lié par covalence avec au moins un cation, lequel cation est choisi parmi H⁺, Na⁺, K⁺, Li⁺, Ca²⁺, Mg²⁺, Sr²⁺, Al³⁺, et les groupes R"A⁺, dans lesquels R" est R ou R' dans lesquels R est l'hydrogène ou un groupe alkyle ou cycloalkyle ayant de 1 à 10 atomes de carbone, et R' est lié par covalence à la molécule d'agent tensioactif et est un groupe alkyle pontant de 1 à 10 atomes de carbone, et A⁺ est N⁺R₃, un ion guanidinium éventuellement substitué avec des atomes d'oxygène, d'azote ou de soufre, ou N⁺B dans lequel B comprend de 3 à 7 atomes choisis parmi les atomes de carbone, d'azote, de soufre et d'oxygène qui complètent le noyau hétérocyclique contenant l'azote; et dans lequel un groupe quelconque R ou R' peut être non substitué ou substitué avec des atomes choisis indépendamment parmi l'oxygène, l'azote ou le soufre; le cation est choisi de telle sorte que la charge nette de la molécule d'agent tensioactif soit neutre; et
(ii) dans lequel le groupe hydrophobe comprend une chaîne hydrocarbonée renfermant au moins 4 atomes de carbone, ou un radical perfluoré renfermant au moins 3 atomes de carbone; et dans lequel l'agent tensioactif a éventuellement au moins une des propriétés suivantes :
(1) un point de fusion supérieur à 20°C; ou
(2) est capable d'être lié par covalence à l'oxyde métallique;
l'agent tensioactif étant présent en quantités nécessaires pour communiquer des propriétés anti-buée aux substrats revêtus sur au moins une surface avec la composition d'enduction et ayant un réseau d'oxyde métallique poreux inorganique d'épaisseur moyenne uniforme, mais non suffisantes pour réduire les propriétés antireflet communiquées par l'oxyde métallique inorganique dont la transparence reste de 3% supérieure à celle du substrat non revêtu, mesurée par le pourcentage de transmission de la lumière à 550 nm.

7. Article comprenant un substrat ayant une surface et une couche d'une composition d'enduction selon la revendication 1 sur au moins une surface du substrat, laquelle composition d'enduction a été séchée.

8. Article selon la revendication 7 caractérisé en outre en ce que la couche de la composition d'enduction a une épaisseur située dans la gamme entre environ 500 et 2500 Å.

9. Protection des yeux comprenant un substrat qui est transparent ou translucide à la lumière visible recouvert avec
(a) un réseau d'oxyde métallique inorganique poreux d'épaisseur moyenne uniforme qui communique des propriétés antireflet au substrat; et caractérisée en ce que ledit substrat est aussi revêtu avec
(b) un agent tensioactif ayant une solubilité dans l'eau de moins de 10 pour cent en poids à 23°C et constitué d'au moins un groupe hydrophobe et d'au moins un groupe anionique hydrophile, dans lequel
(i) le groupe hydrophile anionique comprend un anion choisi parmi -OSO₂O⁻, -SO₂O⁻, -CO₂⁻, (-O)₂P(O)O⁻, -OP(O)(O⁻)₂, -P(O)(O⁻)₂, -P(O⁻)₂, -OP(O⁻)₂, (-SO₂)₂N⁻, -SO₂N(R)⁻, (-SO₂)₂C⁻H et -N⁺(R)₂(CH₂)ₓL', dans laquelle R est l'hydrogène, un groupe alkyle qui est non substitué ou substitué avec des atomes choisis indépendamment parmi l'oxygène, l'azote et le soufre, ou un groupe alkylène-carboxyle, lequel groupe alkyle ou alkylène carboxyle comprend environ de 1 à 10 atomes de carbone; x est 1 à 4; et L' est choisi parmi -OSO₂O⁻, -SO₂O⁻, (-O)₂P(O)O⁻, -OP(O)(O⁻)₂, -P(O)(O⁻)₂, et -CO⁻₂, et dans lequel chaque groupe anionique est associé ou lié par covalence avec au moins un cation, lequel cation est choisi parmi H⁺, Na⁺, K⁺, Li⁺, Ca²⁺, Mg²⁺, Sr²⁺, Al³⁺, et les groupes R"A⁺, dans lesquels R" est R ou R' dans lesquels R est l'hydrogène ou un groupe alkyle ou cycloalkyle ayant de 1 à 10 atomes de carbone, et R' est lié par covalence à la molécule d'agent tensioactif et est un groupe alkyle pontant de 1 à 10 atomes de carbone, et A⁺ est N⁺R₃, un ion guanidinium éventuellement substitué avec des atomes d'oxygène, d'azote ou de soufre, ou N⁺B dans lequel B comprend de 3 à 7 atomes choisis parmi les atomes de carbone, d'azote, de soufre et d'oxygène qui complètent le noyau hétérocyclique contenant l'azote; et dans lequel un groupe quelconque R ou R' peut être non substitué ou substitué avec des atomes choisis indépendamment parmi l'oxygène, l'azote ou le soufre; le cation est choisi de telle sorte que la charge nette de la molécule d'agent tensioactif soit neutre; et
(ii) dans lequel le groupe hydrophobe comprend une chaîne hydrocarbonée renfermant au moins 4 atomes de carbone, ou un radical perfluoré renfermant au moins 3 atomes de carbone;
lequel substrat revêtu lorsqu'il est revêtu sur au moins une surface présente
(1) un diamètre de goutte d'au moins 4 mm à l'essai conforme au Test de Mouillage décrit ici; et
(2) un pourcentage de transmission à 550 nm qui est supérieur d'au moins 3 pour cent à celui du substrat non revêtu.

10. Protection des yeux selon la revendication 9 caractérisée en outre en ce que l'oxyde de métal inorganique est la silice et en ce que l'agent tensioactif est un sulfonate perfluoré.

11. Masque chirurgical comprenant un masque facial et une protection des yeux selon la revendication 9.

12. Procédé destiné à donner à un substrat des propriétés antireflet et anti-buée, le procédé comprenant les étapes consistant :
(a) à fournir un substrat,
(b) à préparer une composition d'enduction
(c) à appliquer la composition d'enduction sur au moins une face du substrat; et
(d) à sécher la composition d'enduction; caractérisé en ce que la composition d'enduction est la composition d'enduction de l'une quelconque des revendications 1 à 6 pour produire un substrat revêtu ayant un réseau d'oxyde métallique inorganique poreux d'épaisseur moyenne uniforme.

13. Procédé destiné à communiquer des propriétés antireflet et anti-buée à un substrat, le procédé comprenant les étapes consistant :
(a) à fournir un substrat,
(b) à préparer une première composition d'enduction comprenant un sol d'oxyde métallique inorganique capable de former un réseau d'oxyde métallique inorganique poreux qui communique à un substrat des propriétés antireflet;
(c) à préparer une seconde composition d'enduction;
(d) à déposer soit la première ou la seconde composition d'enduction sur au moins une face du substrat;
(e) à laisser sécher la couche déposée à l'étape (d);
(f) à déposer la composition d'enduction non déposée à l'étape (d) sur au moins une face du substrat;
(g) à laisser sécher la couche déposée à l'étape (f); caractérisé en ce que la seconde composition de revêtement comprend un agent tensioactif ayant une solubilité dans l'eau de moins de 10 pour cent en poids à 23°C et ayant au moins un groupe hydrophobe et au moins un groupe anionique hydrophile, dans lequel :
(i) le groupe hydrophile anionique comprend un anion choisi parmi -OSO₂O⁻, -SO₂O⁻, -CO₂⁻, (-O)₂P(O)O⁻, -OP(O)(O⁻)₂, -P(O)(O⁻)₂, -P(O⁻)₂, -OP(O⁻)₂, (-SO₂)₂N⁻, -SO₂N(R)⁻, (-SO₂)₂C⁻H et -N⁺(R)₂(CH₂)ₓL', dans laquelle R est l'hydrogène, un groupe alkyle qui est non substitué ou substitué avec des atomes choisis indépendamment parmi l'oxygène, l'azote et le soufre, ou un groupe alkyiène-carboxyle, lequel groupe alkyle ou alkylène carboxyle comprend environ de 1 à 10 atomes de carbone; x est 1 à 4; et L' est choisi parmi -OSO₂O⁻, -SO₂O⁻, (-O)₂P(O)O⁻, -OP(O)(O⁻)₂, -P(O)(O⁻)₂, et -CO⁻₂; et dans lequel attaque groupe anionique est associé ou lié par covalence avec au moins un cation, lequel cation est choisi parmi H⁺, Na⁺, K⁺, Li⁺, Ca²⁺, Mg²⁺, Sr²⁺, Al³⁺, et les groupes R"A⁺, dans lesquels R" est R ou R' dans lesquels R est l'hydrogène ou un groupe alkyle ou cycloalkyle ayant environ de 1 à 10 atomes de carbone, et R' est lié par covalence à la molécule d'agent tensioactif et est un groupe alkyle pontant de 1 à 10 atomes de carbone, et A⁺ est N⁺R₃, un ion guanidinium éventuellement substitué avec des atomes d'oxygène, d'azote ou de soufre, ou N⁺B dans lequel B comprend de 3 à 7 atomes choisis parmi les atomes de carbone, d'azote, de soufre et d'oxygène qui complètent le noyau hétérocyclique contenant l'azote; et dans lequel un groupe quelconque R ou R' peut être non substitué ou substitué avec des atomes choisis indépendamment parmi l'oxygène, l'azote ou le soufre; les cations sont choisis de telle sorte que la charge nette de la molécule d'agent tensioactif soit neutre; et
(ii) dans lequel le groupe hydrophobe comprend une chaîne hydrocarbonée renfermant au moins 4 atomes de carbone, ou un radical perfluoré renfermant au moins 3 atomes de carbone.
